(19) Europäisches Patentamt
European Patent Office
Office européen des brevets

(11) Publication number : **0 587 396 A1**

(12) # EUROPEAN PATENT APPLICATION

(21) Application number : **93307030.2**

(51) Int. Cl.$^5$ : **A61K 31/225**, A61K 31/70

(22) Date of filing : **07.09.93**

(30) Priority : **07.09.92 ZA 926775**
**04.06.93 ZA 933949**

(43) Date of publication of application :
**16.03.94 Bulletin 94/11**

(84) Designated Contracting States :
**AT BE CH DE DK ES FR GB GR IE IT LI LU MC NL PT SE**

(71) Applicant : **VIROSTAT (NA) NV**
**C E B Hellmundweg 11**
**Kralendijk, Bonaire (NA)**

(72) Inventor : **Liebenberg, Roelof Wilke**
**16 Andre Street, Pierneef Park**
**Johannesburg, Transvaal Province (ZA)**
Inventor : **Kruger, Petrus Buys**
**11 Comrie Road**
**Camps Bay, Cape Province (ZA)**
Inventor : **Bouic, Patrick Jacques Desire**
**16 Seventh Avenue**
**Rondebosch East, Cape Province (ZA)**
Inventor : **Albrecht, Carl Franz De Vos**
**15 Antoinette Street, Amanda Glen**
**Durbanville, Cape Province (ZA)**

(74) Representative : **Ellis-Jones, Patrick George Armine**
**J.A. KEMP & CO. 14 South Square Gray's Inn**
**London WC1R 5LX (GB)**

(54) **Use of hypoxoside derivatives for the treatment of viral infections.**

(57) The invention provides compounds, substances and compositions for the treatment of viral infections in human and animal subjects, and medicines and medicaments comprising them. Uses of such compounds, substances and compositions for the treatment of such viral infections, and for use in the preparation of substances and compositions for treating viral infections, in particular HIV infections, are disclosed. Novel compounds, being 1,5-disubstituted pent-4-en-1-yne compounds are disclosed, with methods of making them, and methods of treating viral infections in human or animal subjects. The compounds can be represented by the general formula (1) :

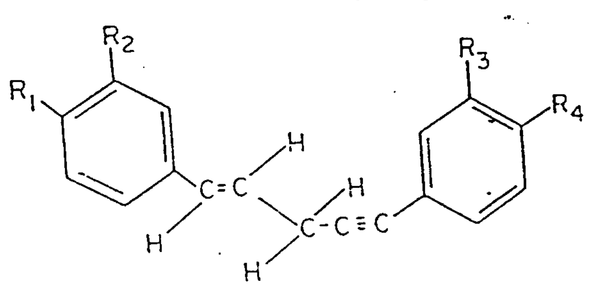

EP 0 587 396 A1

THIS INVENTION relates to the treatment of viral infections. Thus, it relates to a use of a compound, substance or composition in the preparation of a substance or composition for treating a viral infection; to a compound, substance or composition for use in the treatment of a viral infection; to a compound, substance or composition for use as a medicine or medicament for the treatment of a viral infection; to a compound, substance or composition for use as an active therapeutic agent in the treatment of a viral infection; to a compound, substance or composition for use in the preparation of a composition, medicine or medicament for treating a viral infection; to novel compounds suitable for use in the treatment of a viral infection; to a medicine or medicament for the treatment of a viral infection; and to novel methods of preparing compounds and compounds prepared by the method. More particularly, the invention relates to said use, to said compounds, substances or compositions; to said compounds; to said medicine or medicament; and to said methods of preparation, suitable for the treatment of human immunodeficiency virus (HIV) infections, respiratory syncytial virus (RSV) infections and cytomegalo-virus (CMV) infections.

According to the invention there is provided the use, in the preparation of a substance or composition for treating a viral infection in a human or animal subject, particularly an HIV, RSV or CMV infection in a human subject, of a compound, substance or composition comprising at least one compound in accordance with the general formula (1):

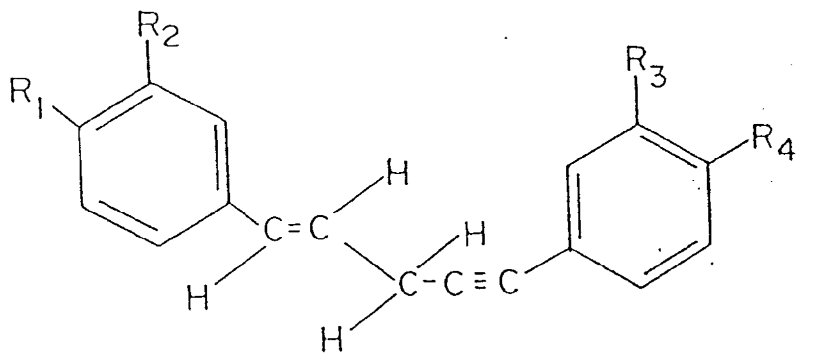

(1)

in which:

$R_1$,$R_2$, $R_3$ and $R_4$ are the same or different and are selected from -H, -OH, A, B, C and D in which :

A =

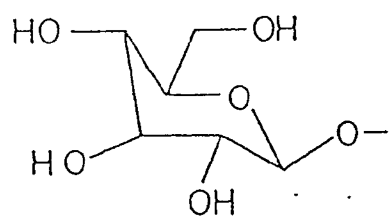

B =

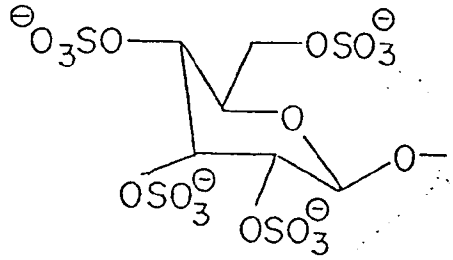

(in which any 1-3 of the $-OSO^{\ominus}_3$ groups is optionally substituted by -OH)

C =

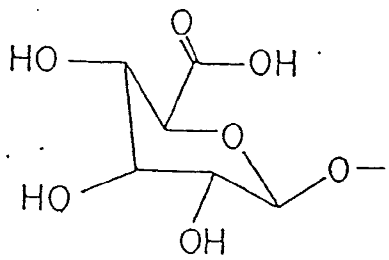

;

and

$$D = -OSO^{\ominus}_3,$$

wherein when $R_1$, $R_2$, $R_3$ and/or $R_4$ represent B or D, a pharmaceutically acceptable cation is also present.

In particular, in each compound of general formula (1), $R_2$ and $R_3$ may be selected from -OH, -H and D; and, in each compound of general formula (1), $R_1$ and $R_4$ may be selected from -OH, A, B, C and D.

More specifically each said compound of general formula (1) may be selected from compounds I - XXVIII having $R_1$, $R_2$, $R_3$ and $R_4$, as set forth in Table 1 hereunder, which are preferred compounds in accordance with said general formula (1). Some of the compounds in Table 1 are present in the plant extracts mentioned hereunder (compounds V - VIII) and others are metabolites thereof (compounds I - IV and IX - XXVIII). Metabolites I - IV are produced in the intestine of the human body, and metabolites IX -XXVIII are produced by phase II biotransformation in the human body. By a phase II biotransformation is meant a biosynthetic reaction whereby a substituent is added to a precursor molecule, which generally renders the precursor molecule more water-soluble and/or biologically less reactive. Hereinbelow it will be appreciated that when reference is made to a compound containing substituent B and/or D, the compound will be present in association with a pharmaceutically acceptable cation.

TABLE 1

| | $R_1$ | $R_2$ | $R_3$ | $R_4$ |
|---|---|---|---|---|
| I | OH | OH | OH | OH |
| II | OH | H | H | OH |
| III | OH | OH | H | OH |
| IV | OH | H | OH | OH |
| V | A | OH | OH | A |
| VI | A | H | H | A |
| VII | A | OH | H | A |
| VIII | A | H | OH | A |
| IX | B | OH | OH | B |
| X | B | H | H | B |
| XI | B | OH | H | B |
| XII | B | H | OH | B |
| XIII | C | OH | OH | C |
| XIV | C | H | H | C |
| XV | C | OH | H | C |
| XVI | C | H | OH | C |
| XVII | D | OH | OH | D |
| XVIII | D | H | H | D |
| XIX | D | OH | H | D |
| XX | D | H | OH | D |
| XXI | C | OH | OH | D |
| XXII | C | H | H | D |
| XXIII | C | OH | H | D |
| XXIV | C | H | OH | D |
| XXV | D | OH | OH | C |
| XXVI | D | H | H | C |
| XXVII | D | OH | H | C |
| XXVIII | D | H | OH | C |

(Any of the hydroxyl groups in compounds IX, XI and XII in Table 1 at the $R_2$ and $R_3$ positions can be substituted by D).

At least one said compound of general formula (1) may form part of an extract from at least one plant species which is a member of the plant family Hypoxidaceae, preferaby forming part of an extract from at least one plant species which is a member of a plant genus selected from the plant genera Hypoxis, Spiloxene, Curculigo and Campynema.

The present invention makes possible a method of treating a viral infection in a human or animal subject, particularly an HIV, RSV or CMV infection in a human subject, which comprises administering to the subject

a substance or composition which comprises an extract from at least one plant species which is a member of the plant family Hypoxidaceae, or which comprises administering to the subject a substance or composition having an active ingredient comprising at least one compound in accordance with the general formula (1) as defined above.

The extract may be a dried extract. The extract may be an extract of at least one plant species selected from the group consisting of *Hypoxis nitida, H. obtusa, H. rigidula, H. villosa, H. interjecta, H. multiceps, H. nyasica* and, particularly *H. rooperi (also known as H. hemerocallidea), H. acuminata* and *H. latifolia (also known as H.colchicifolia* or *H. oligotricha), Spiloxene schlechteri* and hybrids thereof.

As compounds V- VIII occur in the above plant extracts, it follows that, in the above use according to the present invention, each compound of general formula (1) (forming part of said plant extract) may be selected from compounds V-VIII, having $R_1$, $R_2$, $R_3$ and $R_4$ as set forth in Table 1 hereinabove. Naturally, instead, each said compound of the general formula (1) (when it does not form part of a plant extract), may be selected from compounds I-IV and IX-XXVIII, having $R_1$, $R_2$, $R_3$ and $R_4$ as set forth in Table 1 hereinabove. Thus, each compound of general formula (1) may be selected from compounds I-V, or, instead, each compound may be selected from compounds IX-XXVIII.

The above use may be for a viral infection selected from human immunodeficiency virus (HIV) infections, respiratory syncytial virus (RSV) infections and cytomegalo-virus (CMV) infections, particularly an HIV infection.

The compound E-1,5-bis(3'-hydroxy-4'-O-β-D- glucopyranosylphenyl)pent-4-en-1-yne (also known as hypoxoside) is the compound V in accordance with formula (1) in which $R_2$ and $R_3$ are -OH and $R_1$ and $R_4$ are A. The above-mentioned extracts from plants of the family Hypoxidaceae contain one or more compounds I - VIII, particularly V - VII. The isolation of hypoxoside and related 1,5-disubstituted pent-4-en-1-yne compounds from plants of the family Hypoxidaceae is described in Marini-Bettolo *et al*: Tetrahedron, 1982, <u>38</u>, 1683 -1687, and synthesis thereof is described in South African Patent N° 86/4482.

As indicated above, it should be noted that, optionally, any 1 - 3 of the sulphate groups in B can be substituted by -OH; any of the hydroxyl groups in compounds IX, XI and XII can be substituted by D.

The preparation of compounds I - XXVIII is described hereunder.

The plant extract, or said compounds of formula (1), particulary compounds I - XXVIII, may be administered orally, or parenterally.

In the case of parenteral administration, the plant extract or compounds I - XXVIII may be administered intravenously, dissolved in a suitable aqueous solution, such as a saline solution. Intravenous administration may be continuous or intermittent, and may be such as to obtain, on average, blood plasma levels of the compounds I - XXVIII and/or metabolites thereof, such as phase II biotransformation products thereof, of 0.1 - 400 μg/mℓ, such as 25 - 200 μg/mℓ, and preferably 50 - 150 μg/mℓ, e.g. 100 μg/mℓ.

It is expected that, for intravenous administration, the compounds I - XXVIII will be administered in sterile solution at a concentration of 1 - 100 mg/mℓ; typically 50- 100 mg/mℓ, at a rate to achieve steady state plasma levels as described above of said compounds I - XXVIII of up to 400 μg/mℓ blood plasma.

In the case of oral administration, the plant extract or compounds I - XXVIII may be in the form of a tablet, or in the form of a powder or in the form of a solution, and may be contained in a capsule. The solution may be an aqueous solution; and this solution may be a solution of one or more of the compounds I - XXVIII as a mixture in purified form, or a solution of the plant extract. Similarly, the tablet, powder or capsule may comprise the plant extract or one or more compounds I - XXVIII. Each tablet or capsule may contain 20 - 500 mg of one or more of said compounds I - XXVIII as a mixture, preferably 50 - 300 mg, e.g. 200 mg.

Trials with human volunteers have revealed that oral administration of the dried methanolic plant extract at rates up to 3200 mg/day, to provide blood plasma levels in subjects of the order of 200 μg/mℓ, are well tolerated. The only side-effects were limited to a degree of nausea, vomiting and diarrhoea in a small proportion (3 out of 25) of subjects.

In further trials said dried methanolic extract was administered intravenously, after dissolving in a saline solution, to sheep at a dosage rate of 200 mg hypoxoside/kg/day, for at least 30 days and up to 60 days, with no significant drug-related effects other than a reduced mass gain and some discolouration of connective tissue at the site of administration. The method accordingly extends to the parenteral and in particular intravenous administration of the plant extract, particularly the high pressure liquid chromatograph-purified fraction containing compounds V - VIII from the methanolic plant fraction, dissolved in an aqueous solution, preferably a saline solution. Furthermore as compounds IX - XXVIII have been found to be present at combined or total concentration levels in plasma of up to 400 μg/mℓ plasma after oral ingestion by humans of compounds V - VIII, compounds IX - XXVIII are particularly suitable for direct intravenous infusion, dissolved in saline solutions.

According to another aspect of the invention there is provided, as a compound, substance or composition for use in the treatment of a viral infection in a human or animal subject, particularly for the treatment of an

HIV, RSV or CMV infection in a human subject, a compound, substance or composition comprising at least one compound in accordance with the general formula (1) as defined above.

According to another aspect of the invention there is provided a compound, substance or composition for use as a medicine or medicament for the treatment of a viral infection in a human or animal subject, particularly an HIV, RSV or CMV infection in a human subject, the compound, substance or composition comprising at least one compound in accordance with the general formula (1) as defined above.

According to another aspect of the invention there is provided a compound, substance or composition for use as an active therapeutic agent in the treatment of a viral infection in a human or animal subject, particularly an HIV, RSV or CMV infection in a human subject, the compound, substance or composition comprising a compound in accordance with general formula (1) as defined above.

According to another aspect of the invention there is provided a compound, substance or composition for use in the preparation of a composition, medicine or medicament for treating a viral infection in a human or animal subject, particularly an HIV, RSV or CMV infection in a human subject, the compound, substance or composition comprising at least one compound in accordance with the general formula (1) as defined above.

The invention extends further to novel compounds in accordance with general formula (1) as defined above, with the proviso that, when both $R_2$ and $R_3$ are selected from -OH and -H, then $R_1$ and $R_4$ are not both -OH and $R_1$ and $R_4$ are not both A.

According to a still further aspect of the invention there is provided a medicine or medicament for the treatment of a viral infection in a human or animal subject, in particular for the treatment of an HIV, RSV and CMV infection in a human subject, which medicine or medicament comprises, as an active therapeutic agent, at least one compound in accordance with the general formula (1) as defined above, subject to the above proviso.

The medicine or medicament may be in a form selected from tablets, capsules, powders and liquids containing the active therapeutic agent, the tablets and capsules each containing 20-50 mg of the active therapeutic agent and the powders and liquids each containing 4-10 mg/mℓ of the active therapeutic agent.

The invention also extends to novel methods of preparation of the compounds IX -XXVIII as described herein, particularly by the partial synthesis thereof from compounds I - VIII or from derivatives of compounds I - VIII.

Compounds in accordance with general formula (1) selected from compounds I - IV, having $R_1$, $R_2$, $R_3$ and $R_4$ as set forth in Table 1 hereinabove, can be prepared by extracting a compound in accordance with general formula (1) which is selected from compounds V-VIII from at least one plant species which is a member of the plant family Hypixidaeceae, and subjecting said extracted compound to enzymatic hydrolysis, to obtain said compound selected from compounds I-V as a product.

The invention extends further to a method for preparing a compound in accordance with general formula (1) which is selected from compounds IX-XII, having $R_1$, $R_2$, $R_3$ and $R_4$ as set forth in Table 1 hereinabove, the method comprising extracting a compound of general formula (1) which is selected from compounds V-VIII from at least one plant species which is a member of the plant family Hypoxidaceae, and per-sulphating the extracted compound, to obtain said compound selected from compounds IX-XII as a product.

The invention extends further to a method of preparing a compound in accordance with general formula (1) which is selected from compounds XVII - XX, having $R_1$, $R_2$, $R_3$ and $R_4$ as set forth in Table 1 hereinabove, the method comprising extracting a compound in accordance with general formula (1) which is selected from compounds V-VIII from at least one plant species which is a member of the plant family Hypoxidaceae, and subjecting said extracted compound to enzymatic hydrolysis to obtain a compound selected from compounds I-V, the method further comprising sulphating said compound selected from compounds I-IV to obtain said compound selected from compounds XVII-XX as a product.

The invention extends further to a method of preparing a compound of general formula (1) which is compound XIII, having $R_1$, R, $R_3$ and $R_4$ as set forth in Table 1 hereinabove, the method comprising extracting compound V in accordance with general formula (1) from a plant species which is a member of the plant family Hypoxidaceae, subjecting compound V to enzymatic hydrolysis to obtain compound I, and subjecting said compound I to glucuronidation, to obtain said compound XIII as a product.

The invention extends further to a method of preparing a compound of general formula (1) which is selected from compounds I-IV and IX-XXVIII, having $R_1$, $R_2$, $R_3$ and $R_4$ as set forth in Table 1 hereinabove, the method comprising extracting at least one compound in accordance with general formula (1) selected from compounds V-VIII from at least one plant species which is a member of the plant family Hypoxidaceae, causing ingestion of the extracted compound by a human or animal subject, collecting urine from the subject after said ingestion, the urine containing said compound selected from compounds I -V and XIII -XXVIII as a metabolite of the compound selected from compounds V-VIII, and extracting said metabolite from the urine as a product compound.

It will be understood that a compound with an -OH substituent may be converted to a compound with a substituent D and a compound with a substituent A may be converted to a compound with a substituent B by

a standard sulphation procedure well known to a person skilled in the art. Similarly the reverse steps consisting of reverse sulphation are also possible in general.

Naturally, the method may include the step of isolating and purifying each product compound.

The invention extends also to compounds of general formula (1) which are selected from compounds I-IV and IX-XXVIII, having $R_1$, $R_2$, $R_3$ and $R_4$ as set forth in Table 1 hereinabove, when obtained by a method according to the present invention as defined above.

In trials it has been found that, surprisingly, the plant extract and in particular compound V from the plant extract, particularly when per-sulphated, exhibit anti-HIV activities *in vitro.* Furthermore, surprisingly, it has also been found that these compounds, in pure form or as part of the plant extract, when biotransformed (phase II biotransformation) show anti-HIV activities both *in vitro* and *in vivo.* The utility of compounds IX - XXVIII is particularly unexpected and surprising, because phase II (hepatic and/or extrahepatic) biotransformation of compounds usually leads to their biological deactivation. The anti-pathogenic or anti-viral effect of these metabolites is thus completely unexpected. In these trials the treated subjects exhibited a mean decrease in p24 HIV core protein levels and a mean increase in the levels of certain lymphocyte subsets, when compared with the mean values characteristic of HIV infections for untreated subjects.

The invention will now be described, by way of example, with reference to tests which have been conducted to demonstrate anti-viral activity, *in vitro,* against HIV, with reference to clinical trials, with reference to Examples for the preparation of novel compounds IX - XXVIII, and with reference to the accompanying drawings, in which:-

Figure 1 shows a chromatogram plotting mAU (milli-absorbence units) against time (minutes) of a methanolic extract of compounds V-VIII from the corms of *Hypoxis rooperi,* to illustrate the relative amounts of compound V, compound VI and compounds (VII + VIII) in the extract, employing a signal wavelength of 260 nm at a bandwidth of 20 nm, with a reference wavelength of 550 nm at a bandwidth of 50 nm;

Figure 2 shows a chromatogram, similar to Figure 1, of an ethanolic extract of compounds V - VIII from the corms of *H. latifolia,* to illustrate the relative amounts of compound V, compound VI and compounds (VII + VIII) in the extract, the respective aglucones, obtained by enzymic deglucosidation of compounds V - VIII, namely compounds I - IV respectively, being co-cromatographed for compartive purposes, retention times being:

| Compound | Retention Time (minutes) |
|---|---|
| I | 12.18 |
| II | 14.84 |
| (III + IV) | 13.55 |
| V | 8.25 |
| VI | 8.76 |
| (VII + VIII) | 8.50; |

Figure 3 shows a chromatogram, similar to Figure 1, of compounds V - VIII as purified by HPLC from a methanolic extract of *H. rooperi,* retention times being:

| Compound | Retention Times (minutes) |
|---|---|
| V | 8.28 |
| VI | 8.79 |
| (VII + VIII) | 8.54; |

Figure 4 shows a chromatogram, similar to Figure 1, of human serum analysed before ingestion of 1 g of the purified compounds V - VIII whose chromatogram is shown in Figure 3, and, superimposed thereon, a chromatogram of serum from the same subject 90 minutes after ingestion of said compounds, the metabolites of compounds V - VIII comprising the three major peaks of the post-ingestion chromatogram, being analogous to the urinary fractions A, B and C respectively of Figures 5 and 6 hereunder;

Figure 5 shows a chromatogram, similar to Figure 1, of human urine three hours after the ingestion of the

purified compounds V - VIII whose chromatogram is shown in Figure 3 and which illustrates the three urinary fractions A, B and C which are illustrated also in Figure 6;

Figure 6 shows three chromatograms, similar to Figure 1, superimposed to illustrate three fractions, namely fractions A, B and C, of purified metabolites preperatively isolated by HPLC from the urine whose chromatogram is shown in Figure 5, their retention times being:

| Fraction | Retention Time (minutes) |
|---|---|
| A | 7.19 |
| B | 7.90 |
| C | 9.03; |

Figure 7 shows a diode array detector ultraviolet (UV) absorption spectrogram illustrating the absorption spectrum of purified urine metabolite fraction A of Figure 6, being a plot of mAU against wavelength (nm), the spectrogram having absorption
wavelength maxima at 206.5 - 210.5 nm and 258.5 - 262.5 nm respectively, and an absorption wavelength minimum at 230.5 - 234.5 nm;

Figure 8 shows a spectrogram, similar to Figure 7, for the absorption spectrum of fraction B of Figure 6, the spectrogram having absorption wavelength maxima at 206.5 - 210.5 nm and 258.5 - 262.5 nm respectively, and an absorption wavelength minimum at 228.5 - 232.5 nm;

Figure 9 shows a spectrogram, similar to Figure 7, of the absorption spectrum of fraction C of Figure 6, the absorption spectrum having absorption wavelength maxima at 204.5 - 208.5 nm and 256.5 - 260.5 nm respectively, and an absorption wavelength minimum at 228.5 - 232.5 nm;

Figure 10 shows an HPLC chromatogram, similar to Figure 1, of the aconjugates of purified urine fraction A as derived by the hydrolysis of fraction A with β-glucuronidase and aryl-sulphatase at pH 5.5, retention times being:

| Compound | Retention Time (minutes) |
|---|---|
| I | 12.35 |
| II | 14.91 |
| (III + IV) | 13.58 |

correspondence being found between these retention data and those respectively of compound I, compound II and compounds (III + IV), as derived by the action of β-glucosidase on the ehtanolic extract of *H. latifolia* as shown in Figure 2, the absorption spectra of the urine and plant-derived aconjugates also being superimposable;

Figure 11 shows a chromatogram, similar to Figure 10, of said aconjugates derived in similar fashion from purified urine fraction B;

Figure 12 shows a chromatogram, similar to Fifure 10, of said aconjugates derived in similar fashion from purified urine fraction C;

Figure 13 shows a chromatogram, similar to Figure 1, of the products of glucuronidation of compound I, namely the chemically synthesized β-D-glucopyranuronates of compound I obtained by a modified Koenigs-Knorr synthesis, the products being purified by selective solvent partitioning, the chemical β-D-glucuronidation of compound I resulting in two products with retention times and spectra which were coincidental with the urine metabolite fraction A of Figure 5, the spectrogram in question being superimposed in Figure 13;

Figure 14 shows a spectrogram, similar to Figure 7, but with the mAU axis scaled (normalized), for compound I, having absorption wavelength maxima respectively at 210.5 - 214.5 nm, 258.5 - 262.5 nm, and 290.5 - 294.5 nm, and absorption wavelength minima at 234.5 - 238.5 nm and 282.5 - 286.5 nm respectively;

Figure 15 shows a spectrogram, similar to Figure 14, but for compound II, having absorption wavelength maxima respectively at 200.5 - 204.5 nm and 258.5 - 262.5 nm, and an absorption wavelength minimum at 222.5 - 226.5 nm;

Figure 16 shows a spectrogram, similar to Figure 14, but for compounds (III + IV), having absorption wavelength maxima at 202.5 - 206.5 nm and 258.5 - 262.5 nm respectively, and an absorption wavelength minimum at 228.5 - 232.5 nm;

Figure 17 shows spectrogram, similar to Figure 14, but for compound V, having absorption wavelength maxima respectively at 210.5 - 212.5 nm, 254.5 - 258.5 nm and 288.5 - 292.5 nm, and absorption wavelength minima at 232.5 - 234.5 nm and 278.5 - 282.5 nm respectively;

Figure 18 shows a spectrogram, similar to Figure 14, but for compound VI, having absorption wavelength maxima respectively at 200.5 - 204.5 nm and 256.5 - 260.5 nm, and an absorption wavelength minimum at 220.5 - 224.5 nm;

Figure 19 shows a spectrogram, similar to Figure 14, but for compounds (VII + VIII), having absorption wavelength maxima respectively at 204.5 - 208.5 nm and 256.5 -260.5 nm, and an absorption wavelength minimum at 226.5 - 230.5 nm;

Figure 20 shows a spectrogram, similar to Figure 14, but showing superimposed absorption spectra for compounds I and V;

Figure 21 shows a spectrogram, similar to Figure 14, but showing superimposed absorption spectra for compounds II and VI;

Figure 22 shows a spectrogram, similar to Figure 14, but showing superimposed absorption spectra for compounds (III + IV) and (VII + VIII);

Figure 23 shows superimposed chromatograms, similar to Figure 1, of compound II obtained from natural and synthetic sources respectively, the natural compound being obtained by the enzymatic deglucosidation of HPLC-purified compound V obtained from *H. rooperi,* whereas the synthetic compound was synthesized as described by Drewes *et al*: Synthetic Communications, 1990, 20, 1671 - 1679;

Figure 24 shows a spectrogram, similar to Figure 14, of superimposed absorption spectra of the syntehtic and natural versions of compound II whose chromatograms are shown in Figure 23;

Figure 25 shows a chromatogram, similar to Figure 1, for a mixture of compounds IX - XII obtained by the per-sulphation of a mixture of compounds V - VIII;

Figure 26 shows a spectrogram, similar to Figure 7, for the mixture of compounds IX - XII whose chromatogram is shown in Figure 25;

Figure 27 shows a chromatogram, similar to Figure 1, for compounds I - IV as derived from *H. rooperi,* being the products of hydrolysis of compounds V - VIII whose chromatogram is shown in Figure 3, by β-D-glucosidase at pH 5.5, the retention times being:

| Compound | Retention Time (minutes) |
|---|---|
| I | 12.31 |
| II | 14.88 |
| (III + IV) | 13.53; |

Figure 28 shows a chromatogram, similar to Figure 1, of a mixture of compound I and its semisynthetic disulphated product, compound XVII, retention times being:

| Compound | Retention Time (minutes) |
|---|---|
| I | 12.29 |
| XVII | 8.95; |

Figure 29 shows a spectrogram, similar to Figure 14, for compounds I and XVII whose chromatogram is shown in Figure 28, compound I having absorption wavelength maxima respectively at 210.5 - 214.5 nm, 258.5 - 262.5 nm and 290.5 - 294.5 nm and absorption wavelength minima at 234.5 - 238.5 nm and 282.5 - 286.5 nm, while compound XVII had absorption wavelength maxima at 204.5 - 208.5 nm and 254.5 - 258.5 nm and an absorption wavelength minimum at 226.5 - 230.5 nm;

Figure 30 shows a chromatogram, similar to Figure 1, for a mixture of compound II and semi-synthetic compound XVIII which is the disulphated conjugate product of compound II, retention times being:

| Compound | Retention Time (minutes) |
|---|---|
| II | 14.86 |
| XVIII | 9.07; |

Figure 31 shows a spectrogram, similar to Figure 14, for compounds II and XVIII whose chromatogram is shown in Figure 30, compound II having absorption wavelength maxima respectively at 200.5 - 204.5 nm and 258.5 - 262.5 nm, and an absorption wavelength minimum at 222.5 - 226.5 nm, and compound XVIII having absorption wavelength maxima at 198.5 -202.5 nm and 254.5 - 258.5 nm, and an absorption wavelength miminum at 220.5 - 224.5 nm, the absorption spectrum of the di-sulphated product, compound XVIII, displaying a shift to a shorter wavelength, compared with that of compound II;

Figure 32 shows a chromatogram, similar to Figure 1, of a mixture of compounds I-IV and of their semisynthetic di-sulphated conjugates, compounds XVII - XX, obtained by the di-sulphatation of the mixture of compounds I - IV, illustrating the co-elution of the di-sulphates of compounds I - IV, namely compounds XVII - XX, retention times being:

| Compound | Retention Times (minutes |
|---|---|
| I | 12.14 |
| II | 14.80 |
| (III + IV) | 13.51 |
| XVII - XX | 9.00. |

Figure 33 shows bar charts of results of a HIV pilot study showing mean absolute changes of p24 core protein levels in pg/mℓ within groups of treated (invention) and non-treated (control) subjects;

Figure 34 shows bar charts of the pilot study of Figure 33, but for mean absolute changes in cell/μℓ within said treated and non-treated groups for selected lymphocyte subsets; and

Figure 35 shows bar charts of the pilot study of Figure 33, but for mean percentage changes within said groups for said cell subsets.

## EXAMPLE 1

### Summary of the Method for the Analysis, Characterisation and Preparative Isolation of Compounds I-XXVIII

### Preparative Isolation of Compounds

Preparative isolation of compounds was conducted by preparative High Performance Liquid Chromatography.

### Equipment

A Shimadzu (Kyoto, Japan) instrument was used. It consisted of the following components: SCL-8A system controler, 2 x LC-8A mobile phase delivery pumps, SPD-6AV UV-Vis Spectrophotometric detector, FCV-130 AL reservoir inlet controler and FCV-100B fraction collector.

The preparative column (50 x 300 mm) was packed with C8 bonded silica (Partisil Bioprep 20 μm C8 75A) obtained from Whatman (Fairfield, New Jersey, USA).

Operating conditions involved isocratic delivery of mobile phase (15% acetonitrile in water v/v) at a flow rate of 100 mℓ/minute. The detector was set to at 260 nm wavelength, and the fraction collector was set to collect 250 mℓ aliquots. Sample loading was conducted by dissolution of sample (5 g) in water (50 mℓ) which was pumped directly on-column, the column having been pre-loaded with water. Hypoxoside (compound V) and hypoxoside congeners [compounds (VII + VIII) and VI] were eluted in sequence starting at a retention volume of 8.5ℓ, providing fractions enriched with the respective compounds to a purity of 80-100% by mass.

Semi-preparative Isolation and Oualitative Analysis

A standardised High Performance Liquid Chromatography (HPLC) technique (Kruger *et al*: J. Chromatography, 1993, 612, 191-198) was applied to the analysis, characterisation and semi-preparative isolation of all the compounds I-XXVIII.

Equipment

A Hewlett Packard (Waldbron, Germany) instrument was used. It consisted of the following components: HP1090M liquid chromatograph with a binary DR5 solvent delivery system and manual valve injector, HP1040 diode array detector, HP79994A work station, HP310SPU processor with colour monitor, HP9153C 20MB winchester disc drive, AP2225A thinkjet printer and HP7440A X-Y plotter.

The analytical column (4.6 x 250 mm) was packed with end-capped C8 bonded silica of 5 μm regular particle size (Whatman, Maidstone, England) while the guard column (2.1 x 75 mm) was packed with pellicular C18 bonded silica (Whatman). An in-line extraction pre-column (2.1 x 30 mm) as utilised was packed with preparative grade C18 bonded silica of 40 μm particle size (Analytichem International, Harbor City, CA.)

Operating conditions involved a linear solvent gradient programmed at a flow rate of 1.5 mℓ/min starting with mobile phase A (0.05 M $KH_2PO_4$) to which 10% v/v mobile phase B (acetonitrile-isopropanol 80:20 v/v) was added. This mixture was maintained for 1 minute after which a linear gradient of mobile phases A and B were formed which ended in 70% v/v of B after 16 minutes. Column temperature was maintained at 50°C.

The in-line extraction pre-column was loaded with a mixture of sample (dissolved in 50 μℓ of alcohol or in up to 200 μℓ of aqueous phase) and made up to 500 μℓ with an aqueous solution containing guanidinium (8.05 M) and ammonium sulphate (1.02 M). The extraction column was flushed before and after sample introduction by 500 μℓ of aqueous ammonium sulphate (0.5 M). The solutes trapped on the in-line extraction column were then eluted onto the analytical column by the mobile phase upon initiation of the analysis.

Diode array detector parameters were programmed to monitor a signal at 260 nm and also to give an absorption spectrum from 200-400 nm.

EXAMPLE 2

Preparation of Dried Methanolic Extract of *Hypoxis* species

Washed *Hypoxis* species corms are shredded, the shreds placed on drying trays and dehydrated in a convection oven at 70°C over 3.5 hours. The shreds are milled to 200 mesh fineness to provide a fine brown powder (12.5 kg of corms will provide approximately 5.0 kg of dried powder). The dried milled powder (5.0 kg) is extracted with methanol (25 ℓ) at room temperature and with stirring over 30 minutes.

Filtration of the slurry and vacuum evaporation of the clear brown solution provide 1.25 kg of the light brown *Hypoxis* species corm extracts which consistently contains between 45-50% m/m of hypoxoside and its congeners [compounds V-VIII) of which hypoxoside (compound V) is the major (90-95% m/m] component, depending on the particular *Hypoxis* species used.

EXAMPLE 3

Preparative Isolation of Compounds V-VIII (see Figures 1 - 2)

Compound V was isolated from a methanolic extract of *Hypoxis rooperi*, which was then purified by preparative HPLC as described in Example 1 (see Figure 1).

Compounds VI and (VII + VIII) were isolated from an ethanolic extract of *Hypoxis latifolia,* which was then purified by preparative HPLC as described in Example 1. Further purification of these compounds was conducted where necessary by semi-preparative HPLC using the standardised analytical HPLC system described in Example 1 (see Figure 2).

EXAMPLE 4

Isolation of compounds I-IV (see Figure 2)

Compounds I, II and (III + IV) were isolated as the enzymatic hydrolysis products of compounds V, VI and (VII + VIII) respectively as follows:

Compounds V, VI and (VII + VIII) (100 mg of each) were separately dissolved in aqueous acetate buffer (10 mℓ, 0.1 M, pH 5.5) and to each was added β-glucosidase (100 mg). The mixtures were incubated for 4 hours at 37°C, after which the hydrolysis products were extracted with diethyl ether (5 mℓ). The diethyl ether was washed with aqueous sodium bicarbonate (5 ml, 0.5 M) and then dried with anhydrous sodium sulphate. The ether phase was evaporated under a stream of nitrogen gas to yield 5-10 mg of compounds I, II and (III + IV) respectively. Each product was purified by semi-preparative HPLC using the standardised analytical HPLC system described in Example 2. Removal of the HPLC solvent buffer salts from the HPLC fractions was achieved by the standard reverse phase sorption technique and the final powdered products of each obtained by lyophylisation.

EXAMPLE 5

Isolation of Compounds XIII-XXVIII (see Figures 3 - 12)

A methanolic extract of the corms of *Hypoxis rooperi* was purified by preparative HPLC (see Example 1) to yield a mixture containing mainly compound V (90-95% m/m), and to a lesser extent compounds VI and (VII + VIII) (see Figure 3). This product (1 g) was dissolved in water (200 mℓ) and ingested orally as a single dose by an adult male subject. Urine was subsequently collected and treated as follows:

C18 bonded silica sorbent material (200 g, 40 μm) was pre-treated with methanol and water via a column bed. Filtered urine (5 ℓ) was passed through the sorbent bed followed by elution in sequence with water (400 mℓ), aqueous methanol (10% v/v, 400 mℓ), aqueous methanol (30% v/v, 800 mℓ) and finally with methanol (400 mℓ) and followed by water (400 mℓ).

The aqueous methanol fraction (30% v/v, 800 mℓ) was diluted with water (1600 mℓ) and again passed through the sorbent bed which was then eluted with water (400 mℓ) and finally with methanol (400 mℓ).

The final methanol solution was evaporated under vacuum at 50°C to an aqueous residue which was lyophylised to yield a mixture of metabolites XIII-XXVIII and endogenous urine components (1 g). A suitable volume of filtered aqueous solution of this residue (10 mg/mℓ) was injected on to the in-line pre-column of the standardised HPLC system (see Example 1) such that fractions A,B and C (see Figures 5 - 6) were collected at the column outlet. These fractions were rendered free of mobile phase buffer salts by the standard reverse phase sorption technique and lyophylised to dry products, representative of mixtures of compounds XIII-XXVIII.

EXAMPLE 6

Chemical Synthesis of Sulphate Conjugates

Pyridinium sulphate was used as a sulphating agent in the partial synthesis of compounds IX - XII and compounds XVII-XX.

Preparation of Pyridinium Sulphate Reagent

To a mixture of dry ethyl acetate (1 ℓ) and concentrated sulphuric acid (25 ℓ, 0.469 mol) was added slowly, with stirring, dry pyridine (50 mℓ, 0.620 mol). The mixture was cooled and the precipitate of pyridinium sulphate produced was separated from the ethyl acetate by decanting, rinsed with a portion of dry ethyl acetate and vacuum dried at 60°C to yield 85 g (100% yield) of product. The product (85 g, 0.480 mol) was dissolved in dry dimethylformamide (250 mℓ) to provide a 1.921 M pyridinium sulphate solution, herein designated as pyridinium sulphate reagent. The reagent was stored in a hermetically sealed amber bottle over anhydrous calcium sulphate (25 g).

Per-Sulphation of Hypoxoside (Compound V) and Hypoxoside Congeners, [Compounds VI and (VII +VIII)] to Produce Compounds IX, X and (XI + XII) Respectively.

To a solution of hypoxoside (2 g, 0.0033 mol) (see Example 3 and Figure 1) in dry dimethylformamide (130 mℓ) was added pyridinium sulphate reagent (70 mℓ, 0.135 mol), anhydrous calcium sulphate (4 g) and acetic anhydride (13.2 mℓ, 0.14 mol), and the mixture stirred under nitrogen atmosphere at 25°C for 24 hours. The reaction mixture was filtered to a clear solution to which was added, with stirring, a mixture of methanol and 2-propanol (600 mℓ, 1:2 v/v ratio), followed by 2-propanol (250 mℓ). The precipitate so formed was congealed by continued stirring and the solvent mixture removed by decanting. The congealed precipitate was rinsed with 2-propanol and then dissolved in water (40 mℓ). To this solution was added with stirring methanol (100 mℓ)

followed by a solution of sodium hydroxide in methanol (50 mℓ, 1M) to obtain a pH of 7 - 11. The precipitate so formed was isolated by filtration, washed with methanol, and dissolved in water (200 mℓ). This solution was passed through a C18-bonded silica sorbent particle bed (40 μm particle size, bed size: internal diameter 50 mm, length 50 mm) which had been preconditioned with methanol and water. A further 200 mℓ of water was passed through the sorbent bed and combined with the previous eluate, to provide 400 mℓ of clear sodium salt of hypoxoside sulphate solution. This solution was found to be capable of being lyophylised to a dry white product (2 g). It was also found that the sodium salt of hypoxoside sulphate could be precipitated from the eluate by the addition of ethanol (400 mℓ) together with ethyl acetate (1,200 mℓ). The precipitate could be collected by filtration and dried under vacuum to provide a dry white product. The sodium salt of hypoxoside sulphate was readily soluble in water, and, unlike hypoxoside (i.e. compound V), had little or no affinity for a reverse phase chromatography medium such as C18-bonded silica particles, even in the presense of water. Compounds VI and (VII + VIII) were per-sulphated in a similar manner.

Sulphation of Compounds I, II and (III + IV) to Produce Compounds XVII, XVIII, and (XIX + XX) Respectively.

To a solution of compound I (100 mg, 0.00355 mol) (see Example 4 and Figure 2) in dry dimethyl formamide (2.2 mℓ), was added pyridinium sulphate reagent (1.5 mℓ, 0.00288 mol), and acetic anhydride (0.3 mℓ, 0.00318 mol). The mixture was allowed to stand under anhydrous conditions at 90-100°C for 30 minutes, cooled and then poured into cold water (40 mℓ) while stirring. The solution was then passed through a C18-bonded silica sorbent bed (4 g, 40 μm particle size) which had been pre-treated with methanol and then with water.

The following were then passed through the sorbent bed in sequence, water (40 mℓ), aqueous sodium bicarbonate (40 mℓ, 0.1 M), water (40 mℓ) and methanol (10 mℓ) which methanol was separately collected. The methanol eluate was evaporated under vacuum at 50°C to an aqueous residue which was lyophylised to yield 0,07 g of compound XVII. Compounds XVIII and (XIX + XX) were prepared by the same procedure from compounds II and (III + IV) respectively.

EXAMPLE 7

Chemical Synthesis of Compound XIII (see Figure 13)

Compound XIII was derived by the glucuronidation of compound I. The di-β-D-glucuronide of compound I was derived by the classical Koenigs-Knorr reaction, involving the coupling of a protected bromo-glucuronide to the aglycone in the presence of silver carbonate.

The protected bromo-glucuronide, namely methyl (2,3,4-tri-O-acetyl-α-D-glucopyranosyl 1-bromide)uronate,wasprepared according to the method described by Bollenback *et al*: J. Amer. Chem. Soc., 1955, 77, 3310-3315.

The product was not preparatively isolated in view of the trace yield, but was characterised as described in Example 8 hereunder.

EXAMPLE 8

General Procedure for Characterisation of Compounds by the Standardised HPLC Procedure and Enzyme Hydrolysis (see Example 1)

The characterisation of the compounds is based on their absolute chromatographic retention times, their retention times relative to one another, and on their diode array detector absorption spectra as obtained under the standard analytical parameters. Retention times have been found to be reproducible to within limits of about 30 seconds on a range of analytical columns from the same manufacturer. Diode array detector absorption spectra are presented within the limits of resolution of the detector, and the absorption maxima and minima are stated as a wavelength range, ranging from 4-6 nm.

Benzene is used as a reference compound. The analysis of benzene in methanol is characterised as follows with respect to the standardised HPLC method.

| | |
|---|---|
| Retention time | = 13.84 minutes |
| Absorption maxima | = 198.5-202.5 nm |
| | 252.5-256.5 nm |
| Absorption minima | = 220.5-224.5 nm |

Further characterisation of compounds XIII-XXVIII is deduced on the basis of selective enzyme hydrolysis

with β-glucuronidase and aryl sulphatase. D-saccharic acid 1,4-lactone (Sigma 50375)(1 mg/mℓ) and sodium sulphate (0.01 M) were used for the selective inhibition of β-glucuronidase and aryl sulphatase activity respectively where cross reactivity was found in commercial enzyme preparations. Hydrolysis was conducted in acetate buffer (0.1 M pH 5.5).

Characterisation of Compounds I-VIII (see Figures 1 - 2 and Figures 14-22)

An aqueous solution of compound V (see Example 3) prepared from the methanol extract of *Hypoxis rooperi* (see Example 2) was analysed by the standard HPLC method (see Example 1). Compound V (see Figure 1) was isolated as a major component at a retention time of 8.25 minutes, with UV maxima respectively at 210.5-212.5; 254.5-258.5 and 288.5-292.5 nm (see Figure 17). Values quoted by Marini-Bettolo *et al*: Tetrahedron, 1982,38, 1683-1687, are UV maxima respectively at 258, 291, 298 and 310 nm as measured in methanol. Compound V underwent irreversible reaction with alkali and gave a positive reaction for phenolic groups with ferric ferricyanide. Compounds VI and (VII + VIII) are more prevalent in the ethanolic extract of *Hypoxis latifolia* and are therefore better characterised in the isolate obtained from *Hypoxis lafifolia* (see Figure 2).

- Similarity of the UV spectra of compounds VI and (VII + VIII) to the spectrum of compound V (see Figures 17 - 19) suggests that they are structuraly related to compound V.
- Hydrolysis of compounds V, VI and (VII + VIII) by β-D-glucosidase at pH 5.5 yields compounds I, II and (III + IV) respectively, as illustrated in Figure 2.
- The order of elution of compounds V, VI and (VII + VIII) and their aglucones, compounds I, II and (III + IV) respectively remains the same (see Figure 2). From this it is deduced that compounds V, VI and (VII + VIII) differ from each other with respect to substituents on their aglucones. Therefore an increase in the retention time from compound V to compounds (VII + VIII), and then to compound VI and increase in retention time from compound I, and to compounds (III + IV), and then to compound II respectively, will be consistent with a decrease of the number of hydroxyl groups on the aglucones.
- Compound II as derived from *Hypoxis latifolia was* compared with E-1,5-bis(4'-hydroxyphenyl)pent-4-en-1-yne as synthesised by Drewes *et al*: Phytochemistry, 1989, 28, 153-156 with regard to retention time and spectral characteristics coincidence (see Figures 23 -24) confirming the existence of the di-para-hydroxyl groups of compound II.
- Compounds (III + IV) with a retention time intermediate to those of compounds I and II are inferred as having three hydroxyl groups, possible structures being represented by compound III and compound IV, but not yet chromatographically resolved (see Figure 2). The most likely configuration of the tri-hydroxy compound as derived from *Hypoxis rooperi* will be that of compound III since Drewes *et al*: Phytochemistry, 1989, 28, 153-156 provide good evidence that the phenolic ring on the alkene side is a catechol when the compound is derived from *Hypoxis rooperi.*
- Spectral characteristics of compounds I-VIII as illustrated in Figures 14 - 22 respectively are consistent with a common central pent-4-en-1-yne structure.
- The comparative spectra (see Figures 20-22) show that deglucosidation results in a shift to shorter wavelength, under prevailing analytical conditions.
- Retention shifts brought about by hydrolysis and degree of hydroxylation of these chemical derivatives are predictable in terms of reverse phase chromatography retention mechanism.

Characterisation of Compounds IX, X and (XI + XII) (see Figures 25 - 26)

Partial sulphation of compounds V - VIII would result in a large number of hydroxy sulphates, giving a complex elution pattern. However, in the case of total sulphation, three well-defined elution peaks can be expected for each compound. A distinct three-peak pattern is indeed perceptible in Figure 25 and compounds IX, X and (XI + XII) are therefore regarded as the deca-, octa- and nano-sulphates of compounds V, VI and (VII + VIII) respectively. Partial sulphation products have however been prepared by reducing the molar proportion of the sulphating reagent relative to the compound which is sulphated, and complex elution patterns, as expected, were observed.

Compounds IX, X and (XI + XII) are not readily distinguishable in terms of their chromatographic or spectral properties, and a mixture of these compounds is characterised in Figures 25 and 26. Early elution of these compounds (see Figure 25) is consistent with their enhanced water solubility, while they retain the spectral characteristics of compounds V-VIII. It is expected that compounds X and (XI + XII) as prepared by the method in Example 6 will have a similar degree of sulphation to compound IX.

Characterisation of Compounds XIII-XXVIII (see Figures 3 - 12 and 27 -32)

Compounds XIII-XXVIII represent the products of biotransformation of compounds V-VIII. They are found in the serum and urine of human subjects after the ingestion of compounds V-VIII (see Figures 4 - 6). It will be noted from the following observations that certain qualitative and quantitative biochemical events are relevant to the *in vivo* formation of conjugate compounds XIII-XXVIII. This together with confirmation of conjugate type by synthesis and specific enzyme hydrolysis will serve to identify and characterise these compounds.

- Compounds V-VIII have been shown to be hydrolysed in the intestinal tract of human subjects to compounds I-IV, since these aglycones have been detected in human faeces. Compounds I-IV therefore, after absorption from the intestinal tract, are the likely metabolic precursors of compounds XIII-XXVIII.
- From Figures 3 and 27 it will be noted that the relative amounts of compounds V-VIII are representative of the relative amounts of their hydrolysis products, namely compounds I-IV. The relative amounts of compounds I-IV shown in Figure 27 therefore are representative of the relative amounts of these aglucones that are available for intestinal absorption.
- Isolation of urine metabolites by HPLC into fractions A,B and C is illustrated in Figure 6, and the absorption spectra characteristic of compounds I-VIII are retained in the metabolite fractions (Figures 7 - 9). This verifies that the urine metabolite fractions A,B and C have a common origin, namely compounds I, II and (III + IV).
- Complete hydrolysis of metabolite fractions A,B and C by β-D-glucuronidase and aryl-sulphatase at pH 5.5 result in their aconjugate profiles (see Figures 10 - 12). The retention times and absorption spectra of compounds I, II and (III + IV) remain the same, whether they are the products of hydrolysis of plant extracts or of urine metabolite extracts. This re-affirms the type of the conjugate groups: β-D-glucuronates and sulphates of the plant aglucones, namely compounds I, II and (III + IV).
- Selective enzymatic hydrolysis of fractions A,B and C (see Figure 6) suggests that:
  i) Fraction A consists primarily of the di-β-D-glucuronides of compounds I-IV, namely compounds XIII-XVI respectively.
  ii) Fraction B consists primarily of the mono-sulphate-mono-β-D-glucuronide of compounds I-IV, namely compounds XXI-XXIV respectively.
  iii) Fraction C consists primarily of the di-sulphates of compounds I-IV, namely compounds XVII-XX respectively.

Thus the type of the conjugate group seems to be the principal determinant of the retention time of the metabolites in reverse phase chromatography. It is appropriate therefore that Fraction B, the mixed conjugate (that is the mono-β-D-glucuronate mono-sulphate) of compounds I, II and (III +IV), should be intermediate in retention time between their di-β-D-glucuronates (Fraction A) and their di-sulphates (Fraction C).

- The aconjugate profiles of urine fractions A,B and C (see Figures 10 - 12) do not contain the same relative amounts of compounds I-V as the material ingested, (see Figures 3 and 27) suggesting selective absorption of compounds II and (III + IV) over compound I, and/or suggesting the biotransformation of compound I to compounds (III + IV) and then to compound II, with subsequent conjugation.
- The chemical sulphation of compound I (see Figures 28 - 29), compound II (see Figures 30 - 31) and a mixture of di-sulphated compounds I-IV (see Figure 32) result in products with retention times in the region of the retention time of urine metabolite Fraction C (see Figure 6) shown by the action of aryl-sulphatase to comprise primarily the di-sulphates of compounds I-IV.

The hydrolysis of the chemically synthesised di-sulphates by aryl-sulphatase results in their respective aconjugates, compounds I, II and (III + IV).

- The chemical glucuronidation of compound I (see Figure 13) results in two products with retention times and absorption spectra that correspond with the two urine metabolite peaks of Fraction A (see Figures 5 - 6). The semi-synthetic glucuronides of compound I are hydrolysed by β-D-glucuronidase. The two glucuronate products of compound I may represent glucuronidation at either the meta- or para position - this is untested.
- All conjugates of compounds I, II and (III + IV) show a shift in their absorbance spectra to a lower wavelength as compared to their aconjugates under the prevailing analytical parameters. This is independent of whether they are glucosides, glucuronides or sulphates, whether semi-synthetic or biologically derived. These comparative spectra may be seen in Figures 20 - 22, 29 and 31.

Metabolic and/or synthetic conjugation of aconjugtes I and (III + IV) may take place at hydroxyl groups at the meta-position of the phenyl rings, resulting in many more combinations of conjugated products. It is believed however that para-substitution is preferred due to chemical considerations.

EXAMPLE 9

Compounds I - XXVIII were subjected to *in vitro* testing for inhibition of human immunodeficiency virus type 1 (HIV-1) proliferation as follows:

Human peripheral blood lymphocytes (PBL's) obtained by density gradient centrifugation of blood from healthy volunteers at a blood bank were pooled and used for the propagation of HIV-1 *in vitro.*

The PBL's were polyclonally activated with phytohaemagglutinin (PHA) and subjected to quality controls whereby their viability was tested with Trypan blue exclusion and the expression of activation antigens, $CD4^+$ and $CD25^+$ was measured by immunofluorescence. Prior to adding the test substance, the activated PBL's were exposed to a standardized titre of HIV-1 virus and after incubation for 60 minutes the cells were washed twice and plated into microtitre plates at a concentration of $2X10^6$ cells/well. The medium contained interleukin-2 and in control wells the virus produced between 50 and 100 ng/mℓ of p24 core protein on day 4. The test substance was added to these cultures either in water or dimethyl sulphoxide (DMSO) depending on solubility characteristics. The final DMSO concentration, when used, was always 1% v/v.

HIV replication was measured as the production of p24 core protein and the production of HIV RNA on days 2, 3 and 4, or on days 3, 4, 5 and 6. Core protein p24 was measured using 'Sandwich ELISA' technology using *inter alia* a monoclonal antibody against p24 core protein. The HIV RNA was measured using a specific labelled DNA probe against HIV RNA and the process of hybridization.

Results were expressed as percent inhibition of HIV-1 p24 core protein production or RNA production on days 3, 4 or 6.

Possible toxic effects of the test substances were measured by viability testing using Trypan blue or a proliferation assay using3-(4,5-dimethylthiazol-2-yl)-2,5-diphenyl-2*H*-tetrazolium bromide (MTT) on day 4 or day 6 after initiation of the experiment (Borenfreund *et al*: Toxicol. In Vitro, 1988, 2, 1-6).

It is to be noted that DMSO at 1% by v/v does not inhibit HIV-1 replication.

The toxicity of the drug was expressed as a percentage inhibition of lymphocyte proliferation as measured with MTT and compared with controls receiving the virus but no drug.

In each test, cells were also exposed to HIV-1 and 3'-azido-3'-deoxythymidine (also known as Zidovudine or AZT) at 100, 10, 1 and 0.1 ng/mℓ. This was a positive control and compounds tested were compared to the inhibitory effect of 10 ng/mℓ AZT.

In all of the following Tables, percentage inhibition refers to inhibition of p24 core protein production and HIV RNA production relative to infected controls. Percentage AZT inhibition refers to inhibition caused by 10 ng/mℓ AZT at the end of the experiment, i.e. after 4 or 6 days.

CRUDE EXTRACTS

A dried methanolic extract of *Hypoxis rooperi,* (see Figure 1), was dissolved in DMSO and added to test cells at a final concentration of 70 μg/mℓ (DMSO final concentration was 1% v/v). After 4 days of incubation p24 core protein production was inhibited by 100% and HIV RNA production by 85%. Cell proliferation was 76% of that of the control. AZT at 10 ng/mℓ caused 100 % inhibition of p24 core protein production and 96% of HIV RNA production.

PURIFIED COMPOUND V

In order to ascertain which component of the crude methanolic extract was involved in HIV-1 proliferation inhibition, HPLC-purified hypoxoside, (see Figure 3), was tested *per se* dissolved in water. The following results were obtained after 6 days using a standard titre of HIV-1:

TABLE 2

| Compound | μg/mℓ | % inhibition:p24 core protein | % inhibition: HIV-RNA | Proliferation as a percentage of control value |
|---|---|---|---|---|
| V | 50 | 99 | 100 | 82 |
| V | 25 | 9 | 83 | 93 |
| V | 12.5 | 5 | 65 | 94 |
| V | 6.25 | 5 | 42 | 94 |

At 10 ng/mℓ AZT inhibited p24 core protein production by 20% and HIV-RNA production by 68%.

Using the same sample of compound V but lowering the viral titre ten-fold, the following results were obtained:

TABLE 3

| Compound | μg/ℓ | % inhibition:p24 core protein | % inhibition:HIV-RNA | Proliferation as a percentage of control value |
|---|---|---|---|---|
| V | 50 | 100 | 99 | 82 |
| V | 25 | 90 | 97 | 93 |
| V | 12.5 | 0 | 11 | 94 |
| V | 6.25 | O | 0 | 94 |

At 10 ng/mℓ AZT inhibited p24 core protein production and HIV-RNA by 99%.

## COMPOUNDS V -VIII

In a further test, compounds V-VIII, as isolated from *Hypoxis latifolia,* (see Figure 2), were tested, as dissolved in water, and results obtained after 3 and 6 days of incubation were the following:

TABLE 4 (After 3 days)

| Compounds | μg/mℓ | % inhibition:p24 core protein | % inhibition:HIV-RNA | Proliferation as a percentage of control value |
|---|---|---|---|---|
| V-VIII | 100 | 100 | 100 | 50 |
| V-VIII | 50 | 100 | 100 | 52 |

At 10 ng/mℓ AZT caused 50% inhibition of p24 core protein production and 46% inhibition of HIV-RNA production.

TABLE 5 (After 6 days)

| Compounds | μg/mℓ | % inhibition:p24 core protein | % inhibition:HIV-RNA | Proliferation as a percentage of control value |
|---|---|---|---|---|
| V-VIII | 100 | 100 | 99 | 50 |
| V-VIII | 50 | 100 | 100 | 52 |

At 10 ng/mℓ AZT caused 50% inhibition of p24 core protein production and 46% inhibition of HIV-RNA production.

COMPOUNDS IX-XII

Hypoxoside (compound V) and its structurally related compounds were sulphated to yield compounds IX-XII, see Figure 25) and tested for inhibition of HIV-1 proliferation after 6 days, yielding the following results:

TABLE 6 (Standard viral titre)

| Compound | µg/mℓ | % inhibition:p24 core protein | % inhibition:HIV-RNA | Proliferation as a percentage of control value |
|---|---|---|---|---|
| IX-XII | 50 | 100 | 100 | 93 |
| IX-XII | 25 | 100 | 100 | 88 |
| IX-XII | 12.5 | 29 | 87 | 90 |
| IX-XII | 6.5 | 8 | 54 | 98 |

At 10 ng/mℓ AZT caused 20% inhibition of p24 core protein production and 68% inhibition of HIV-RNA production.

TABLE 7 (Ten-fold dilution of standard viral titre)

| Compound | µg/mℓ | % inhibition:p24 core protein | % inhibition:HIV-RNA | Proliferation as a percentage of control value |
|---|---|---|---|---|
| IX-XII | 50 | 100 | 100 | 93 |
| IX-XII | 25 | 100 | 100 | 88 |
| IX-XII | 12.5 | 97 | 98 | 90 |
| IX-XII | 6.25 | 28 | 23 | 98 |

At 10 ng/mℓ AZT caused 99% inhibition of p24 core protein and HIV-RNA production.

COMPOUND XVII

Compound I was sulphated to yield compound XVII (see Figures 28 -29) which was tested for inhibition of HIV-1 proliferation *in vitro,* yielding the following results:

TABLE 8 (3 days incubation)

| Compounds | µg/mℓ | % inhibition:p24 core protein | % inhibition:HIV-RNA | Proliferation as a percentage of control value |
|---|---|---|---|---|
| XVII | 100 | 88 | 93 | 80 |
| XVII | 50 | 47 | 68 | 84 |

At 10 ng/mℓ AZT caused 50% inhibition of p24 core protein production and 46% inhibition of HIV-RNA production.

TABLE 8 CONTINUED - 6 days incubation

| Compounds | µg/mℓ | % inhibition:p24 core protein | % inhibition:HIV-RNA | Proliferation as a percentage of control value |
|---|---|---|---|---|
| XVII | 100 | 100 | 100 | 94 |
| XVII | 50 | 87 | 91 | 96 |

At 10 ng/mℓ AZT caused 96% inhibition of p24 core protein production and 92% inhibition of HIV-RNA production.

A mixture of compounds I-IV was sulphated to give compounds XVII-XX respectively (see Figure 32) and incubated for 6 days yielded the following results.

TABLE 9

| Compounds | µg/mℓ | % inhibition:p24 core protein | % inhibition:HIV-RNA | Proliferation as a percentage of control value |
|---|---|---|---|---|
| XVII-XX | 100 | 100 | 100 | 80 |
| XVII-XX | 50 | 100 | 100 | 86 |

At 10 ng/mℓ AZT caused 96% inhibition of p24 core protein production and 92% inhibition of HIV-RNA production.

COMPOUND XVIII

Compound II was sulphated to give compound XVIII (see Figures 30 - 31) and yielded the following results after 6 days.

TABLE 10

| Compounds | µg/mℓ | % inhibition:p24 core protein | % inhibition:HIV-RNA | Proliferation as a percentage of control value |
|---|---|---|---|---|
| XVIII | 100 | 100 | 100 | 50 |
| XVIII | 50 | 79 | 87 | 63 |

At 10 ng/mℓ AZT caused 96% inhibition of p24 core protein production and 92% inhibition of HIV-RNA production.

*IN VIVO*-PRODUCED METABOLITES OF COMPOUNDS V-VIII

Orally ingested hypoxoside (compound V) is converted to compound I in the large intestine by bacterial β-glucosidase. Compound I is excreted in the faeces but is also absorbed and converted by phase II biotransformation to sulphate and glucuronic acid conjugates (fractions A, B and C in Figure 6). Fraction A, containing primarily the diglucuronides of compounds I-IV, i.e. compounds XIII-XVI, yielded the following results:

TABLE 11

| Compounds | μg/mℓ | % inhibition:p24 core protein | % inhibition:HIV-RNA | Proliferation as a percentage of control value |
|---|---|---|---|---|
| 4 days incubation | | | | |
| XIII-XVI | 100 | 45 | 20 | 97 |
| XIII-XVI | 100 | 57 | 31 | 97 |

At 10 ng/mℓ AZT caused 100% inhibition of p24 core protein production and 97% inhibition of HIV-RNA production.

FRACTIONS A - C

Fractions A, B and C of urinary metabolites of compounds V-VIII (i.e. compounds XIII-XVI (fraction A); compounds XXI-XXVIII (fraction B) and compounds XVII-XX (fraction C) (see Figures 5 - 6)) were tested and yielded the following results after 3 days of incubation.

TABLE 12

| Compounds | μg/mℓ | % inhibition:p24 core protein | % inhibition:HIV-RNA | Proliferation as a percentage of control value |
|---|---|---|---|---|
| 3 days incubation Fraction A (Figure 6) | | | | |
| XIII-XVI | 100 | 80 | 97 | 80 |
| XIII-XVI | 50 | 25 | 73 | 86 |
| Fraction B (Figure 6) | | | | |
| XXI-XXVIII | 100 | 68 | 81 | 78 |
| XXI-XXVIII | 50 | 36 | 71 | 73 |
| Fraction C (Figure 6) | | | | |
| XVII-XX | 100 | 86 | 100 | 81 |
| XVIII-XX | 50 | 67 | 81 | 79 |

At 10 ng/mℓ AZT caused 50% inhibition of p24 core protein production and 46% inhibition of HIV-RNA production.

Fraction B of the urinary metabolites of compounds V-VIII (see Figure 6) was sub-fractionated into two fractions. Fraction V1 is the ascending half of fraction B and fraction V2 is the descending half of fraction B. These fractions were tested individually for inhibition of HIV replication and yielded the following results over 4 and 6 days of incubation.

TABLE 13

| Compounds | μg/mℓ | | % inhibition:p24 core protein | % inhibition:HIV-RNA | Proliferation as a percentage of control value |
|---|---|---|---|---|---|
| 4 days incubation | | | | | |
| XXI-XXVIII | V1 | 100 | 100 | 33 | ND |
| XXI-XXVIII | V2 | 100 | 100 | 100 | ND |
| 6 days incubation | | | | | |
| XXI-XXVIII | V1 | 200 | 84 | 70 | ND |
| XXI-XXVIII | V2 | 200 | 100 | 100 | ND |

(ND = not determined)

At 10 ng/mℓ AZT inhibited p24 core protein production and HIV-RNA production on days 4 and 6 by 100%.

EXAMPLE 10

MATERIAL AND METHODS:

Subjects:

All patients entered into the study were HIV-positive as determined by a commercial enzyme-linked immunosorbent assay (ELISA) and after a positive ELISA result had been confirmed by the Western blotting technique.

The cohort studied consisted of 18 subjects of both sexes of whom 7 volunteerd to act as a treated group (*Hypoxis* species extract, see Example 2), while the remaining 11 subjects served as a control, non-treated group. No other anti-retroviral agents were used by any subject in the study.

Venous blood samples (clotted and whole EDTA) were drawn at the first visit (baseline) and again at monthly intervals.

Capsules:

Each capsule contained 200 mg *Hypoxis* species extract (about 100 mg of compounds V - VIII) and the patients were advised to ingest 2 such capsules 3 times daily, i.e. a total of 1200 mg/day of extract was ingested.

Blood Analysis:

Clotted Blood:

This specimen of blood was used to quantitate serum HIV antigenemia by means of a commercial capture ELISA kit. The incubation conditions used included the pretreatment of each serum sample with glycine-HCl buffer in order to dissociate immune complexes. This ensured that so-called total p24 core protein was measured and not only that which remains in circulation, uncomplexed by anti-p24 core protein antibodies.

The results were expressed as absolute pg/mℓ serum as determined from a calibration curve.

EDTA whole blood:

Whole blood was used to identify and quantify lymphocyte subsets by monoclonal antibodies. Briefly, 100 μℓ of blood was incubated with mixtures of monoclonal antibodies labelled with different fluorochromes as follows:

CD3-FITC/CD4-PE
CD3-FITC/CD8-PE
CD3-FITC/CD19-PE

CD3-FITC/CD16 + CD56-PE
CD8-FITC/HLA-Dr-PE

These mixtures detect the following cellular subsets: T-helpers; T-suppressors; B-cells; NK-cells and activated suppressor cells respectively.

After a 20 minute incubation, red blood cells were lysed with a lysing solution (FACS lysing buffer) and the cells were pelleted and washed once with phosphate buffered saline solution (PBS). The cells were fixed in 500 μℓ PBS containing 0.5% v/v formaldehyde.

Cell fluorescence was assessed using a FACScan cytometer. FL1 (fluoroscein) was measured using a 530/30 nm band pass filter and the FL2 (phycoerythrin) with a 585/42 nm band pass filter. The analysis was conducted using the Simulset software programme which automatically sets up a lymphocyte gate by the exclusion of granulocytes and monocytes from the analytical gate.

All the results were expressed as percentage positive lymphocytes i.e. cells which co-expressed CD3 and CD4 or CD3 and CD8, etc. In this way, CD4-positive monocytes were excluded from the calculation of $CD4^+$ T-cells. Similarly, $CD8^+$ natural killer (NK) cells were excluded from the $CD3^+$ $CD8^+$T-suppressor subset.

Absolute cell numbers were calculated from basic haematology conducted on an aliquot of the whole blood. From the percentage lymphocyte differential counts, absolute values for positive cells could be calculated. This was conducted for all subsets analysed.

Changes observed between the baseline and follow-up values within subsets were analysed in the following manner:

The absolute value for a particular subset for a patient at baseline = X
The same parameter measured at the first follow-up = X'
Hence the change in this parameter = X' - X
A positive value for X' - X indicates an increase in the parameter measured. Conversely, a negative value indicates a decrease in the parameter between the follow-up value and that of baseline.

The mean change in this parameter for each group was calculated for all the subjects in the group and the results expressed as either the mean absolute change or as the mean percentage change relative to the baseline value.

RESULTS:

Serum Viral Antigenemia (p24 core protein Levels)

Serum p24 core protein determinations revealed that the treated group (n = 7) showed a mean decrease of -13.8 pg/mℓ when compared to the control, non-treated cohort (n = 11) whose mean p24 core protein levels rose by 17.9 pg/mℓ over the same period of 1 month. Similarly, when the p24 core protein levels of the 2-month follow-up were analysed, the treated group (n = 6) exhibited a mean increase of 2.5 pg/mℓ which compared favourably with the mean increase of 31.4 pg/mℓ in the non-treated group (n = 4). These results are represented graphically in Figure 33.

It should be noted that, in four of the treated patients whose follow-up periods were longer, the p24 core protein levels are non-detected by the method employed while no such decreases or reversion to negativity was highlighted in any of the non-treated subjects. On the contrary, the non-treated subjects exhibited ever-increasing p24 core protein levels with time.

Lymphocyte Subsets:

As shown in Table 1, the patients in the treated group (n = 7) showed very favourable increases in all the lymphocyte subsets analysed. Indeed, the mean increase in total lymphocyte counts after a month was accompanied by general increases in $CD3^+$ cells (T cells), T-helper cells ($CD4^+$), T-suppressor cells ($CD8^+$), B cells and NK cells, as well as the fraction of $CD8^+$ cells expressing markers of cellular activation ($CD8^+$ Dr cells). These mean increases in the cellular subsets measured compare favourably with published results of clinical trials conducted with other anti-retroviral agents such as AZT (as mentioned in Example 9) and Didanosine. Indeed, Fisch *et al* (New Eng. J. Med., 1990, 323, 1009-1014) reported a transient mean increase of 25 $CD4^+$ cells/$mm^3$ of blood in their groups treated with standard doses (250 mg/4 hours) and reduced doses (100 mg/4 hours) of AZT for 30 months.

On the other hand, the non-treated group (n = 11) showed deteriorating immune parameters over the same period of time (Table 14). As can be seen, most cellular subsets exhibited decreases after a month of follow-up which appears to confirm the decline in immune status in these subjects as highlighted by the p24 core protein levels reported above.

## TABLE 14

## Mean Absolute Changes in the Cellular Subsets after 1 Month Follow-Up. Comparison of the Groups

Values are expressed as absolute cells/$\mu\ell$ blood.

| | NON-TREATED SUBJECTS (n = 11) | TREATED SUBJECTS (n = 7) |
|---|---|---|
| Total Lymphocytes | -122 | + 144 |
| $CD3^+$ Cells | -109 | +100 |
| $CD4^+$ Cells | -17 | + 36 |
| $CD8^+$ Cells | -89 | + 69 |
| B Cells | +23 | +21 |
| NK Cells | -34 | +36 |
| Activated $CD8^+$ Cells | -51 | +52 |

Positive results indicate an increase relative to the baseline value while a negative value indicates a decrease relative to the baseline value.

These differences between the groups are not only evident when one compares the mean absolute changes, but are also reflected in the mean percentage changes relative to the baseline values. For ease of comparison, these data are represented in Figure 34.

Similar results were obtained with the 2 month follow-up parameters. As can be seen from Table 15, the non-treated group (n = 4) exhibited ever decreasing numbers of $CD4^+$, B cells and NK cell numbers. Other cellular subsets showed a slight increase. However, in the treated group, all the parameters measured showed constant, significant increases for both the mean absolute changes as well as in the mean percentage changes (Table 15). Once again, the mean absolute changes observed in our treated group compare favourably with published data from other clinical trials conducted with AZT. These results are shown in Figure 35.

TABLE 15

Mean Absolute Changes in the Cellular Subsets after a 2 Month Follow-Up Comparison of the Groups

Values are expressed as absolute cells/ℓ blood

| | NON-TREATED SUBJECTS (n = 4) | TREATED SUBJECTS (n = 5) |
|---|---|---|
| Total Lymphocytes | +28 | +218 |
| $CD3^+$ Cells | +12 | +114 |
| $CD4^+$ Cells | -42 | +38 |
| $CD8^+$ Cells | +17 | +80 |
| B cells | -10 | +62 |
| NK Cells | -13 | +97 |
| Activated $CD8^+$ Cells | +28 | +14 |

Positive values indicate an increase relative to the baseline value while a negative value indicates a decrease relative to the starting value.

From Example 10 an extract from *Hypoxis* species has been shown to have anti-HIV activity *in vitro* in that it inhibits both HIV replication (p24 core protein production) and HIV RNA production.

The results are promising in that they show that subjects ingesting 1,200 mg of *Hypoxis* species extract/day exhibit improved immune status after 1 month and also after 2 month on the treatment. The immune parameters measured include: increased numbers of T-cells (both T-helper and T-suppressor cells); B-cells and NK cells. In parallel, there was a substantial decrease and retardation of p24 core protein production as measured in the sera of the subjects.

On the other hand, control, non-treated subjects exhibited ever-deteriorating immune parameters and increasing p24 core protein serum levels over the same period. These subjects showed continuous loss of their $CD4^+$ cells as well as other cellular subsets as shown in other anti-HIV clinical trials (Foulds *et al*: Drugs, 1992, 44, 94- 116 and Prince *et al*: J.Acq. Immuno. Def., 1991, 4, 1227-1232).

## EXAMPLE 11

### Inhibition of Respiratory Syncytial Virus (RSV) by Compound XVIII

Compound XVIII was added to Hep-2 cells growing as slope cultures in plastics tubes to give final concentrations respectively of 50, 100 and 200 μg/mℓ. The cultures were then respectively inoculated with a high standard viral dose, one tenth (1/10) of said standard dose, and one hundredth (1/100) of said standard dose of respiratory syncytial virus (RSV). After four days of incubation at 33°C the cells were visually inspected using an inverted phase contrast microscope and scored for the extent of syncytium formation, i.e. single cells containing three or more nuclei, as follows:

a = 0 - 25%, b = 25 - 50%, c = 50 - 75% and d = 75 - 100% of cells having three or more nuclei.

The results obtained are shown in Table 16 hereunder:

TABLE 16

| Extent of Syncytium Formation in the Presence and Absence of Compound XVII after 4 days of Incubation | | | |
|---|---|---|---|
| Conditions of Incubation | Viral Dose | | |
| | Full | 1/10 | 1/100 |
| Virus control | d | c | b |
| Virus plus 50 µg/mℓ | d | c | a |
| Virus plus 100 µg/mℓ | c | a | Nil |
| Virus plus 200 µg/mℓ | Nil | Nil | Nil |

EXAMPLE 12

Effect of Compound XVII on Cytomegalo-virus (CMV)

Compound XVII was added to primary foetal human fibroblasts in tissue culture, to yield final concentrations respectively of 50, 100 and 200 µg/mℓ. The cultures were then respectively inoculated with a high standard viral dose, with one tenth (1/10) of said standard dose and with one fiftieth (1/50) of said standard dose of cytomegalo-virus (CMV) and incubated for four days.

The cytopathic effect of CMV was determined by microscopic visual inspection of the cell cultures, in terms of the extent of gross swelling of the cells, where:
a = 0 - 25%, b = 25 - 50%, c = 50 - 75% and d = 75 - 100% of the cells affected by gross swelling.

The results obtained are shown in Table 17 hereunder.

TABLE 17

| Effect of Compound XVII on the Extent of the Cytopathic Effect of Cytomegalo-Virus after 4 days of Incubation | | | |
|---|---|---|---|
| Conditions of Incubation | Viral Dose | | |
| | Full | 1/10 | 1/50 |
| Virus control | c | b+ | a |
| Virus plus 50 µg/mℓ | bc | b | a |
| Virus plus 100 µg/mℓ | bc | ab | a- |
| Virus plus 200 µg/mℓ | Nil | Nil | Nil |

At 200 µg/mℓ 5 out of 8 cultures showed signs of toxicity due to the compound.

## Claims

1. Use, in the preparation of a substance or a composition for treating a viral infection in a human or animal subject of a compound of general formula (1):

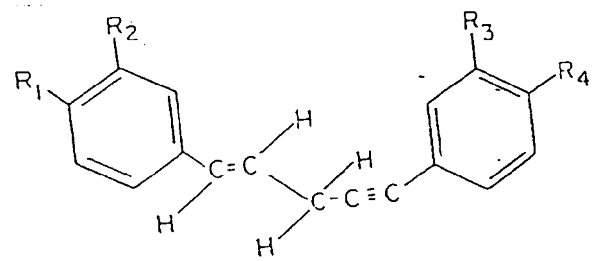

in which:

$R_1$, $R_2$, $R_3$ and $R_4$ are the same or different and are selected from -H, -OH, A, B, C and D in which:

A =

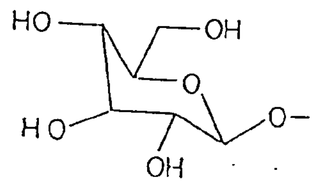

B =

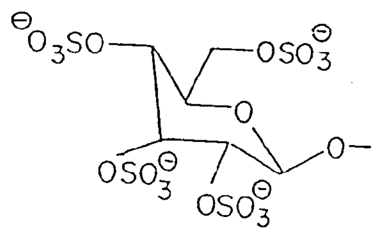

(in which any 1-3 of the $-OSO_3^-$ groups is optionally substituted by -OH)

C =

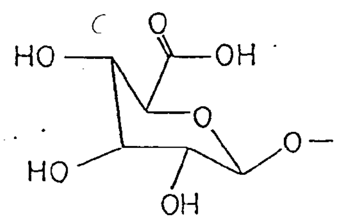

D = $-OSO_3^-$;

wherein when $R_1$, $R_2$, $R_3$ and/or $R_4$ represent B or D, a pharmaceutically acceptable cation is also present.

**2.** Use as claimed in claim 1, wherein the compound of general formula (1) is selected from compounds I - XXVIII, having $R_1$, $R_2$, $R_3$ and $R_4$ as set forth in the following table:

| | $R_1$ | $R_2$ | $R_3$ | $R_4$ |
|---|---|---|---|---|
| I | OH | OH | OH | OH |
| II | OH | H | H | OH |
| III | OH | OH | H | OH |
| IV | OH | H | OH | OH |
| V | A | OH | OH | A |
| VI | A | H | H | A |
| VII | A | OH | H | A |
| VIII | A | H | OH | A |
| IX | B | OH | OH | B |
| X | B | H | H | B |
| XI | B | OH | H | B |
| XII | B | H | OH | B |
| XIII | C | OH | OH | C |
| XIV | C | H | H | C |
| XV | C | OH | H | C |
| XVI | C | H | OH | C |
| XVII | D | OH | OH | D |
| XVIII | D | H | H | D |
| XIX | D | OH | H | D |
| XX | D | H | OH | D |
| XXI | C | OH | OH | D |
| XXII | C | H | H | D |
| XXIII | C | OH | H | D |
| XXIV | C | H | OH | D |
| XXV | D | OH | OH | C |
| XXVI | D | H | H | C |
| XXVII | D | OH | H | C |
| XXVIII | D | H | OH | C |

3. Use as claimed in claim 1 wherein in the compound of general formula (1), when both $R_2$ and $R_3$ are selected from -OH and -H, $R_1$ and $R_4$ are not both -OH or both A.

4. Use as claimed in claim 3 wherein in the compound when $R_1$, $R_2$ and $R_3$ represent -H, $R_4$ does not represent -H.

5. Use as claimed in claim 3 or 4 wherein in the compound substituents $R_2$ and $R_3$ are selected from -OH, -H and D.

6. Use as claimed in claim 3, 4 or 5 wherein in the compound substituents $R_1$ and $R_4$ are selected from -OH, A, B, C and D.

7. Use as claimed in claim 3, 4 or 5 wherein the compound is selected from compounds IX to XXVIII with substituents $R_1$, $R_2$, $R_3$ and $R_4$ as listed in the following table:

| | | | | |
|---|---|---|---|---|
| IX | B | OH | OH | B |
| X | B | H | H | B |
| XI | B | OH | H | B |
| XII | B | H | OH | B |
| XIII | C | OH | OH | C |
| XIV | C | H | H | C |
| XV | C | OH | H | C |
| XVI | C | H | OH | C |
| XVII | D | OH | OH | D |
| XVIII | D | H | H | D |
| XIX | D | OH | H | D |
| XX | D | H | OH | D |
| XXI | C | OH | OH | D |
| XXII | C | H | H | D |
| XXIII | C | OH | H | D |
| XXIV | C | H | OH | D |
| XXV | D | OH | OH | C |
| XXVI | D | H | H | C |
| XXVII | D | OH | H | C |
| XXVIII | D | H | OH | C |

8. A compound as defined in any one of claims 4 to 7.

9. A process for the preparation of a compound as defined in claim 1 which process comprises extracting a compound of general formula (1):

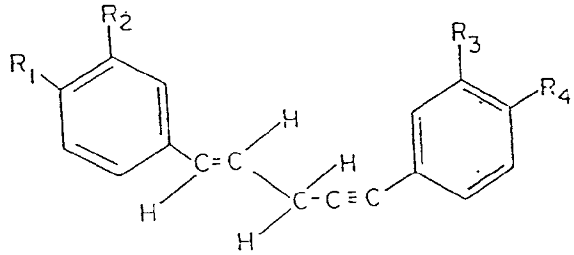

wherein $R_1$ and $R_4$ represent A, and $R_2$ and/or $R_3$ represent -H or -OH, from a plant species which is a member of the plant family Hypoxidaceae and then performing any one of the following reactions:
(a) per-sulphating the extracted compound to obtain a compound where $R_1$ and $R_4$ both represent B;

(b) subjecting the extracted compound to enzymatic hydrolysis to obtain a compound where $R_1$ and $R_4$ both represent -OH and then sulphating the thus obtained compound to yield a compound where $R_1$ and $R_4$ both represent D;

(c) subjecting the extracted compound where $R_2$ and $R_3$ both represent -OH to enzymatic hydrolysis and then subjecting the thus obtained compound to glucuronidation to yield a compound where $R_1$ and $R_4$ both represent C; or

(d) causing ingestion of the extracted compound by a human or animal subject, collecting urine from the subject after ingestion and extracting a compound where $R_1$ and/or $R_4$ represent C or D from the urine.

**10.** A pharmaceutical composition which comprises a compound as defined in any one of claims 3 to 7, in association with a pharmaceutically acceptable carrier or coating.

**11.** A compound as defined in any one of claims 3 to 7 for use in the treatment of a viral infection in a human or animal subject.

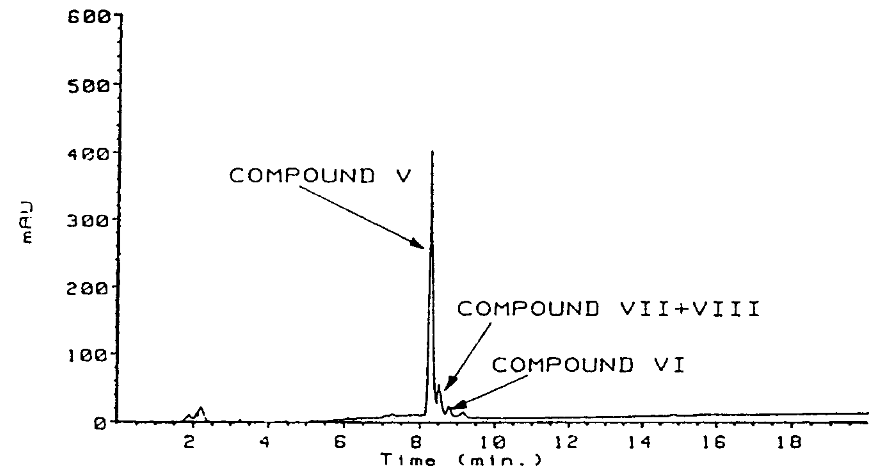


FIG 1

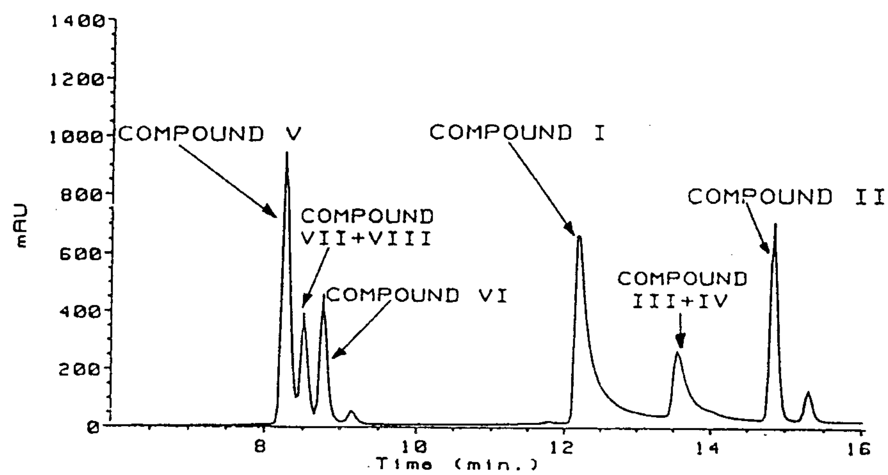


FIG 2

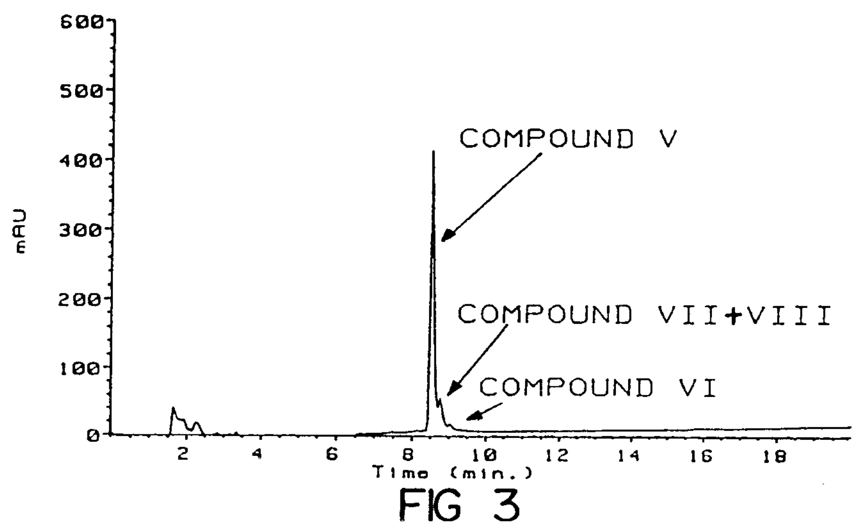


FIG 3

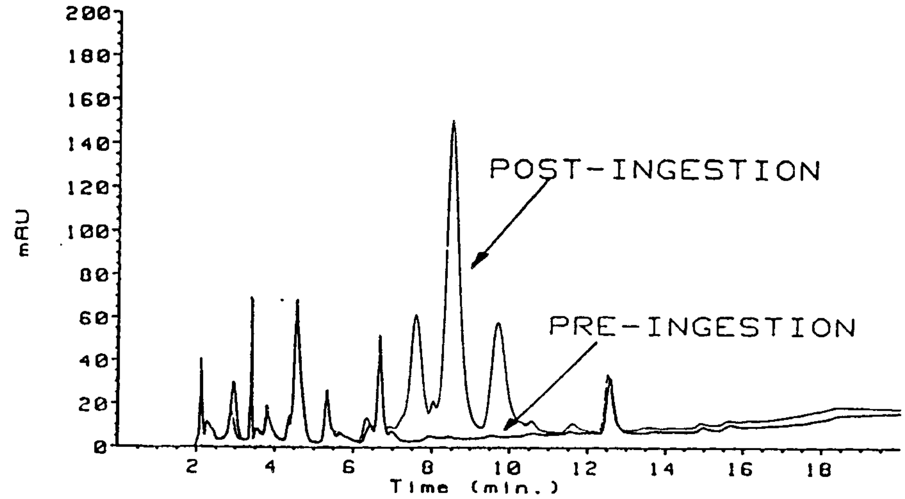


FIG 4

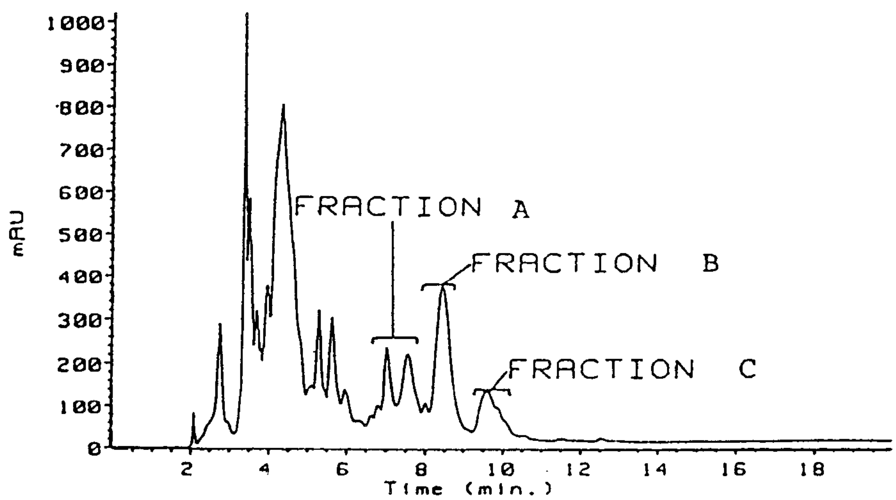


FIG 5

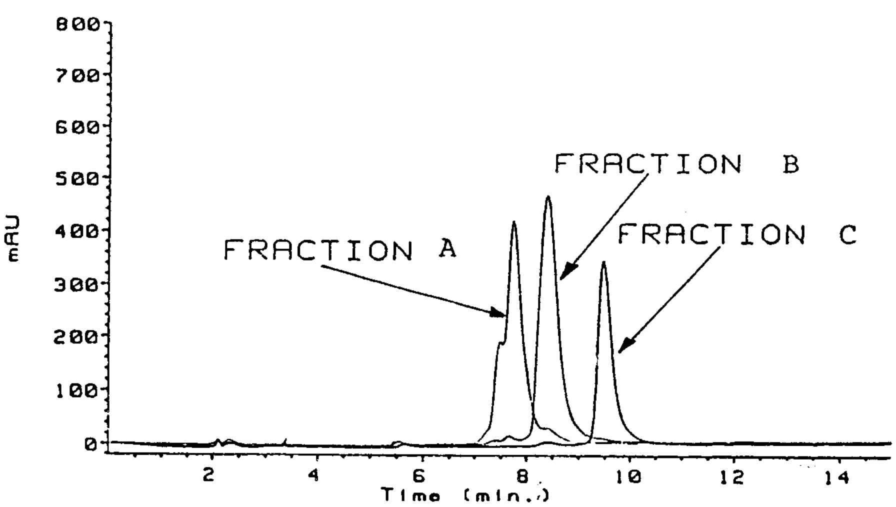


FIG 6

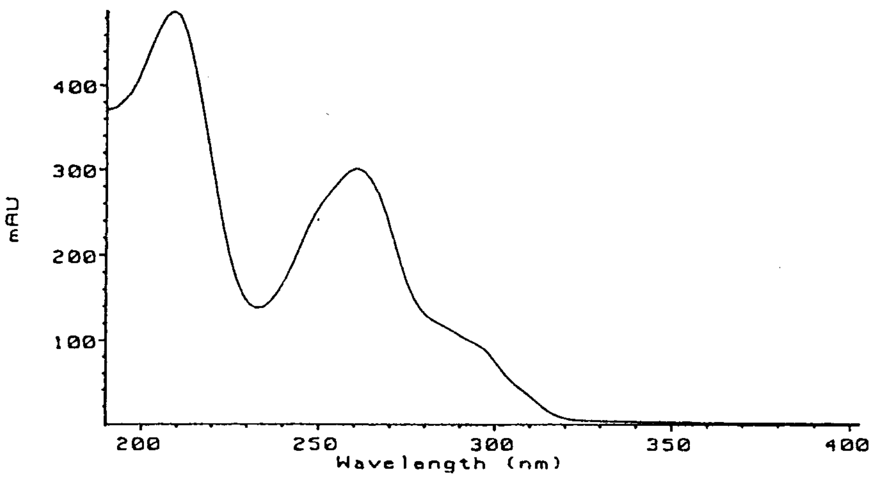


FIG 7

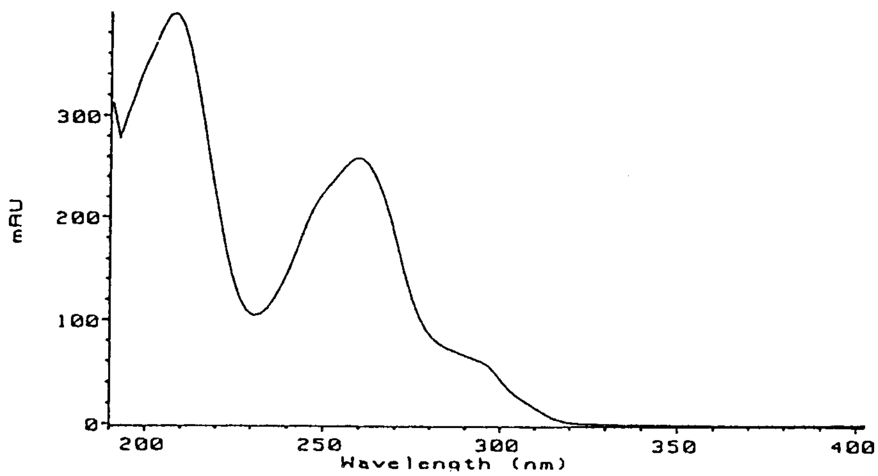


FIG 8

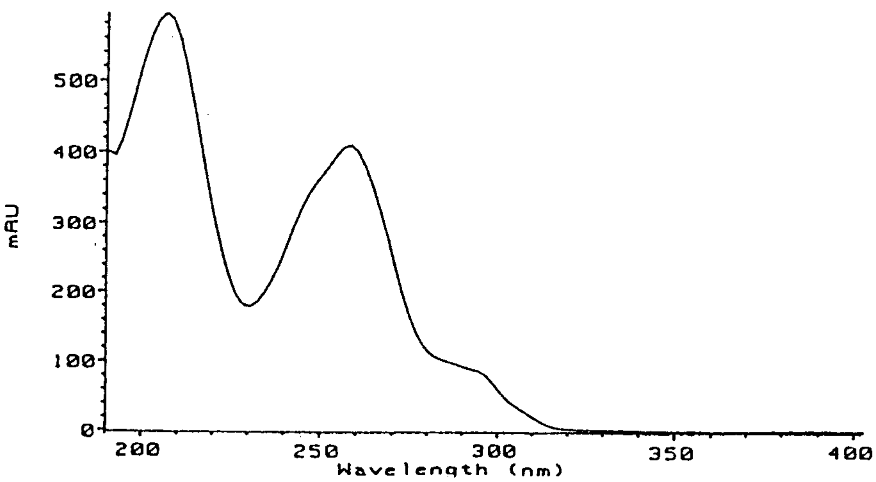


FIG 9

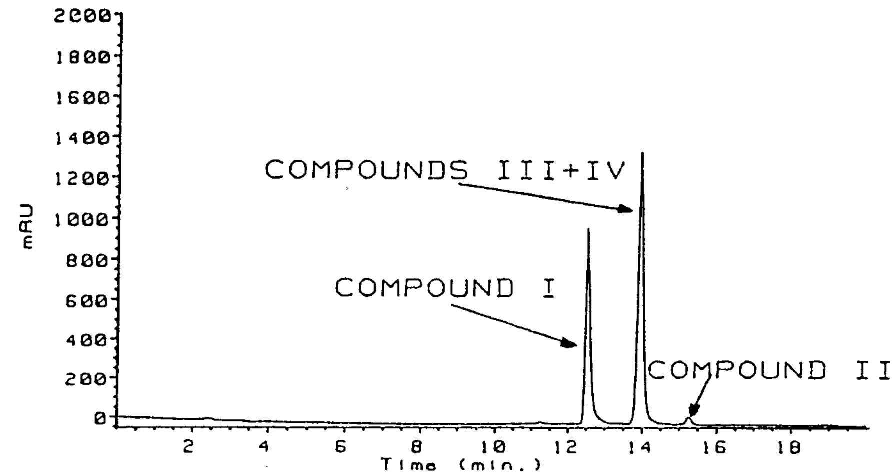


FIG 10

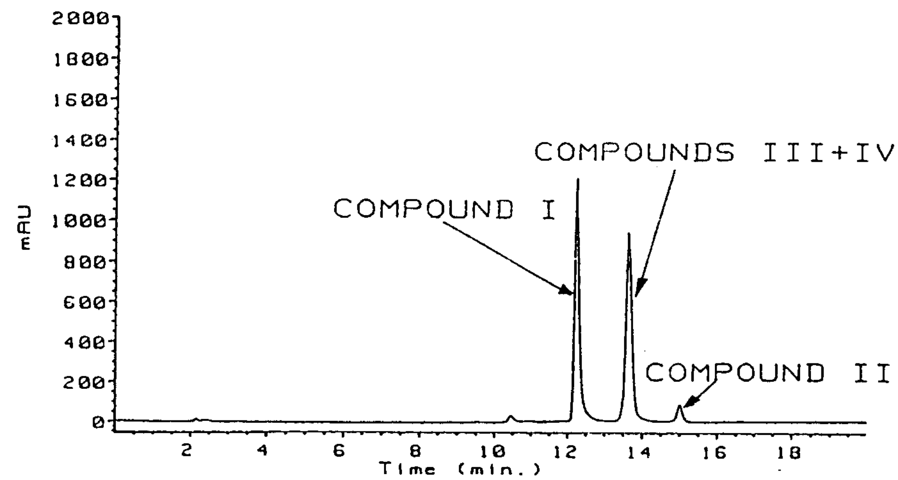


FIG 11

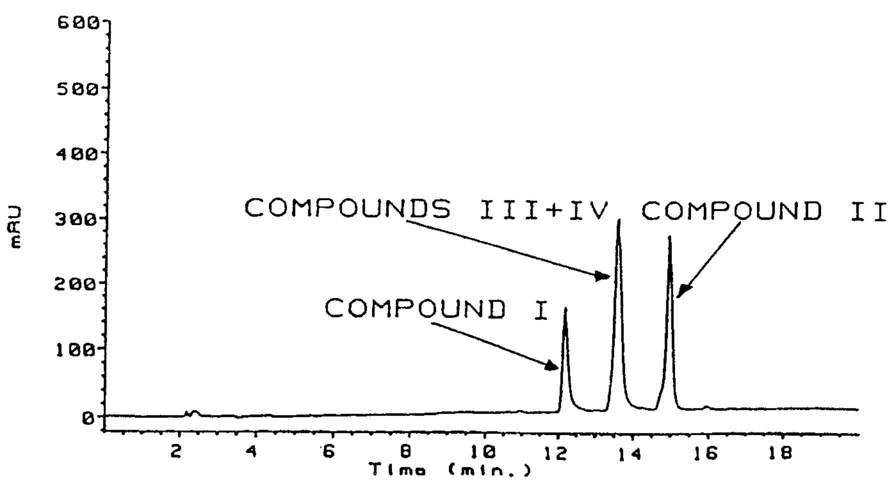


FIG 12

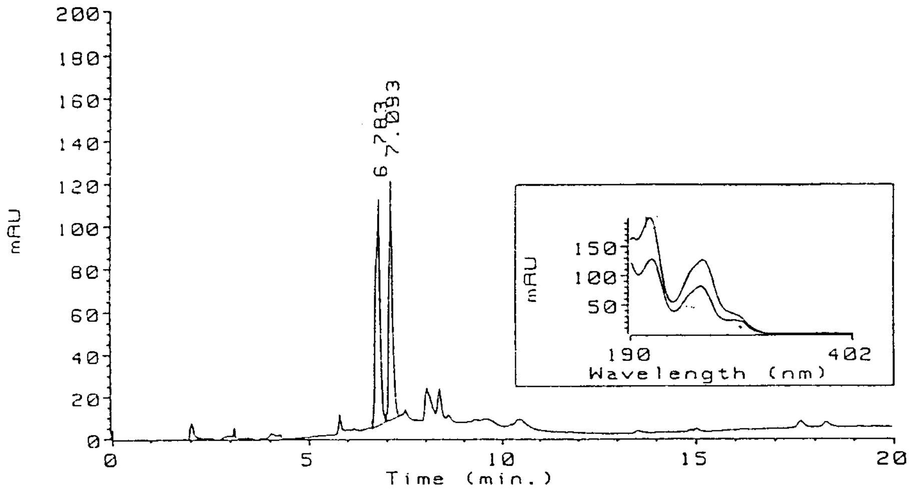


FIG 13

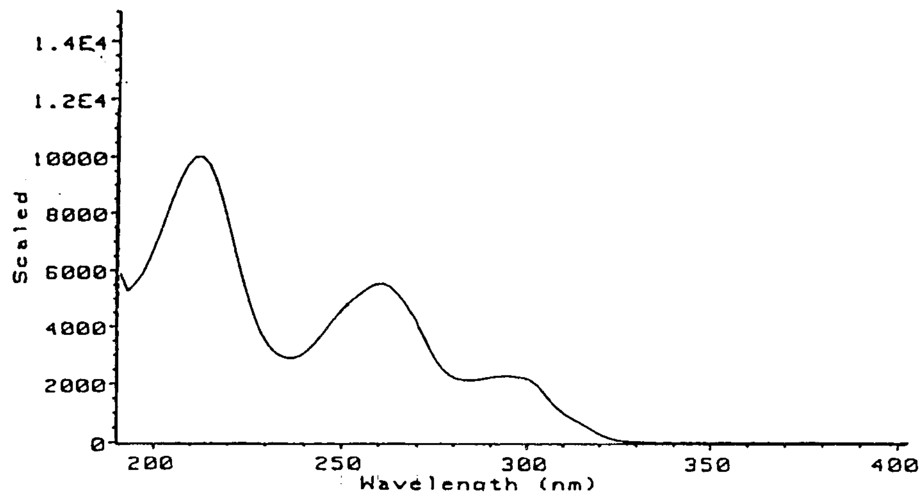


FIG 14

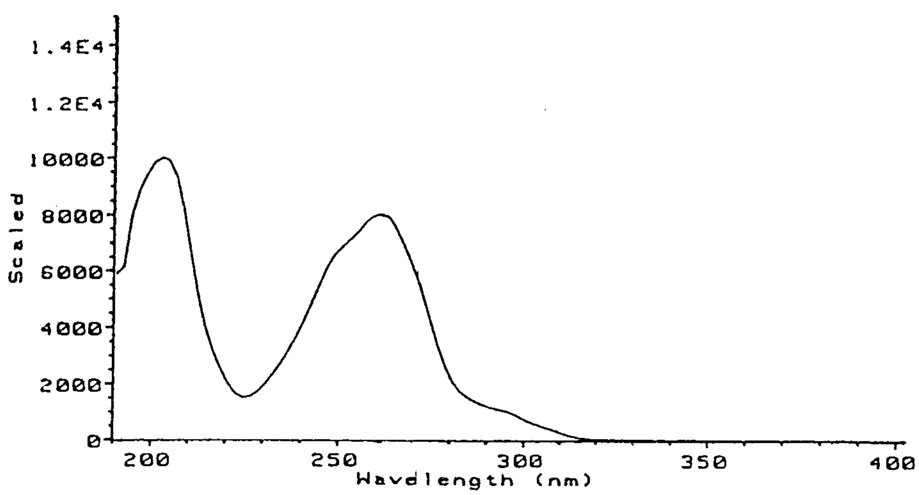


FIG 15

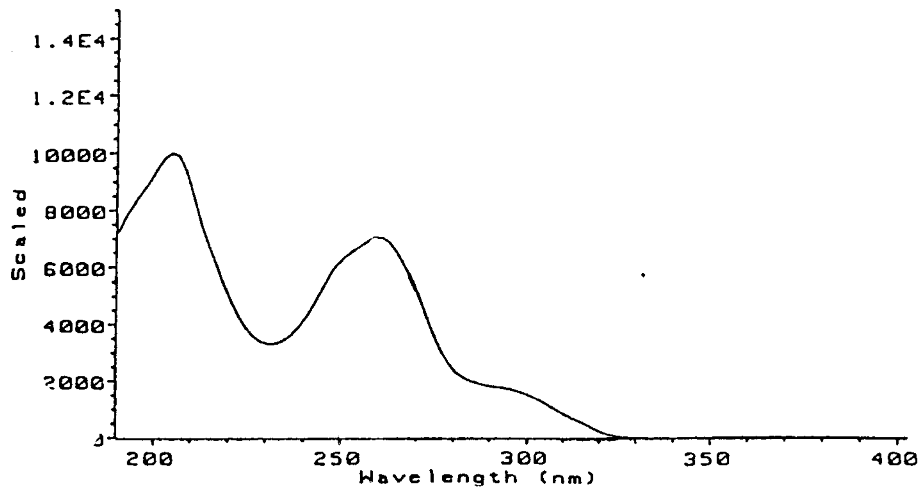


FIG 16

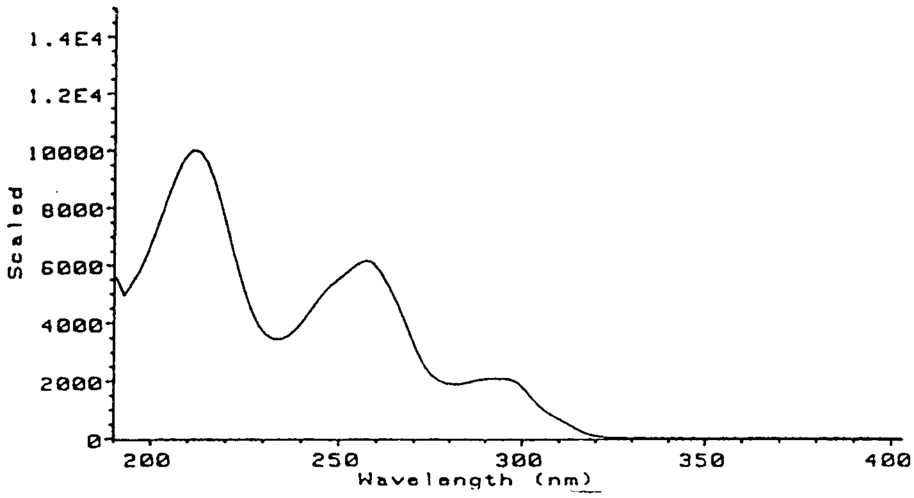


FIG 17

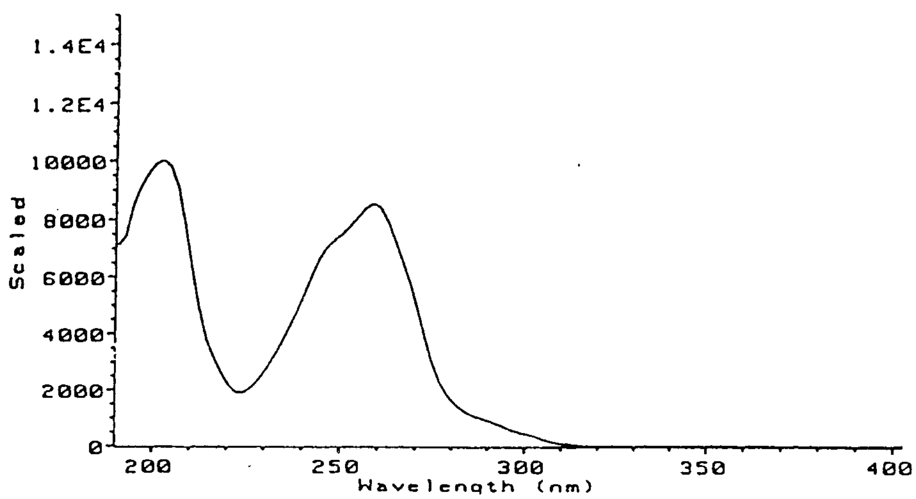


FIG 18

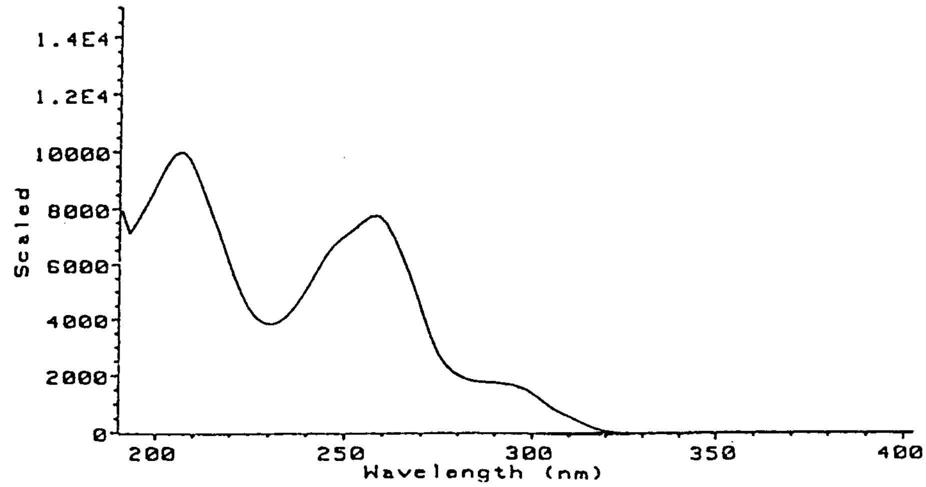


FIG 19

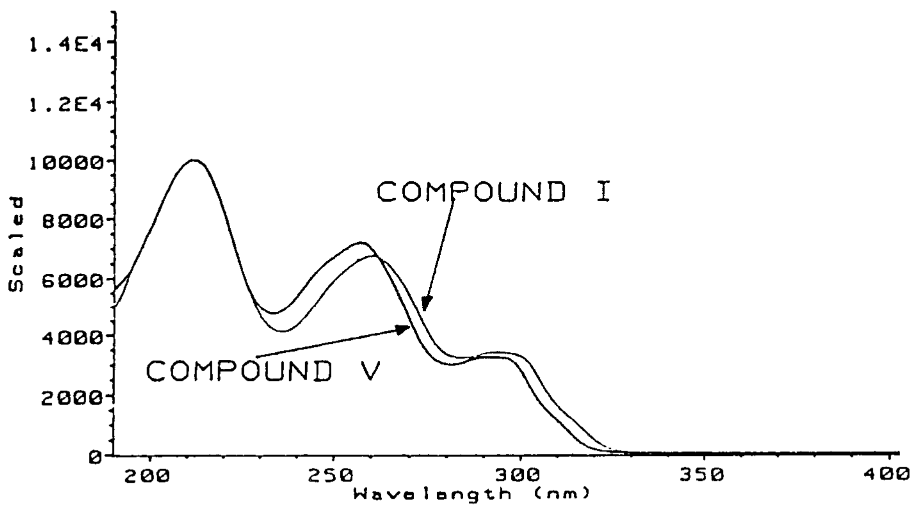


FIG 20

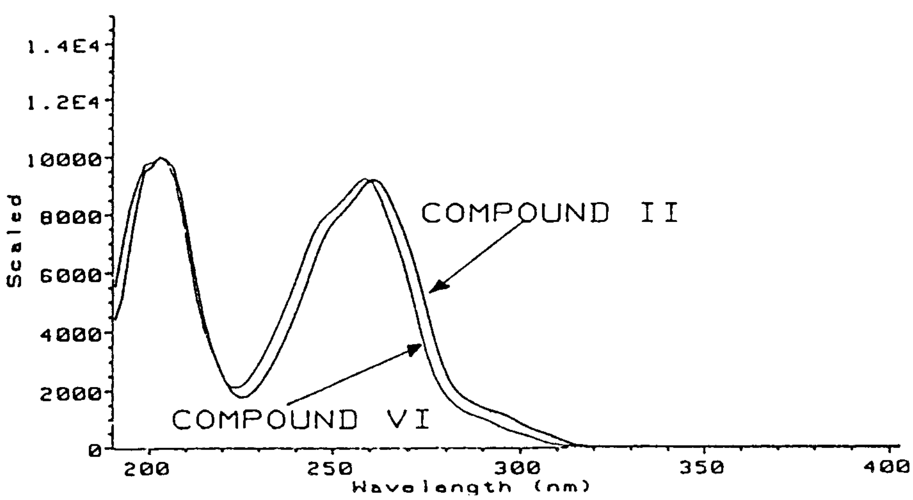


FIG 21

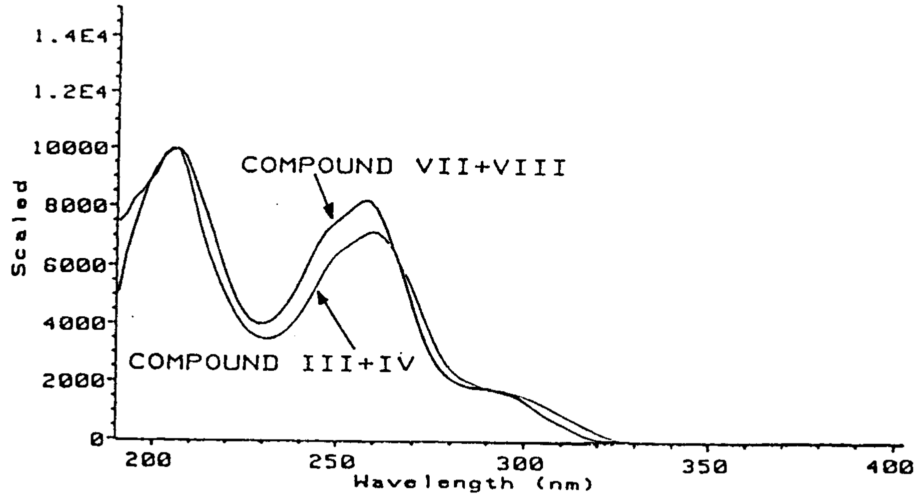


FIG 22

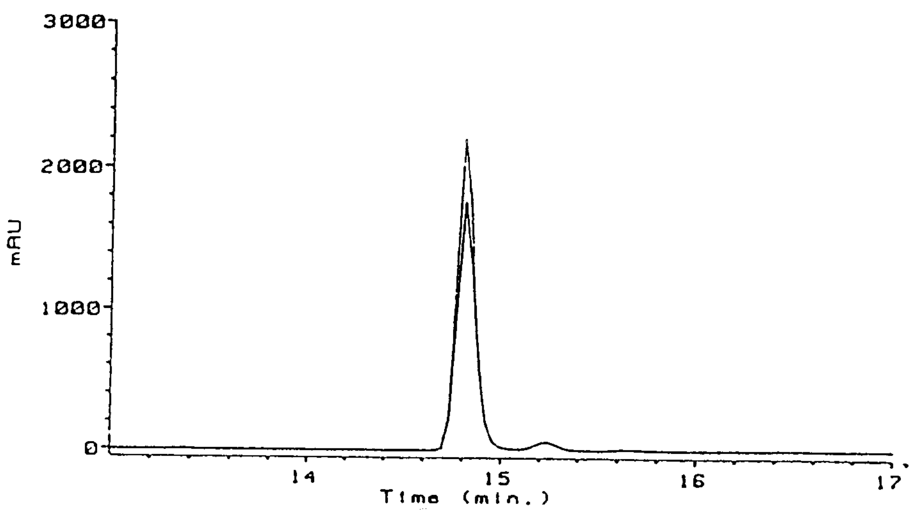


FIG 23

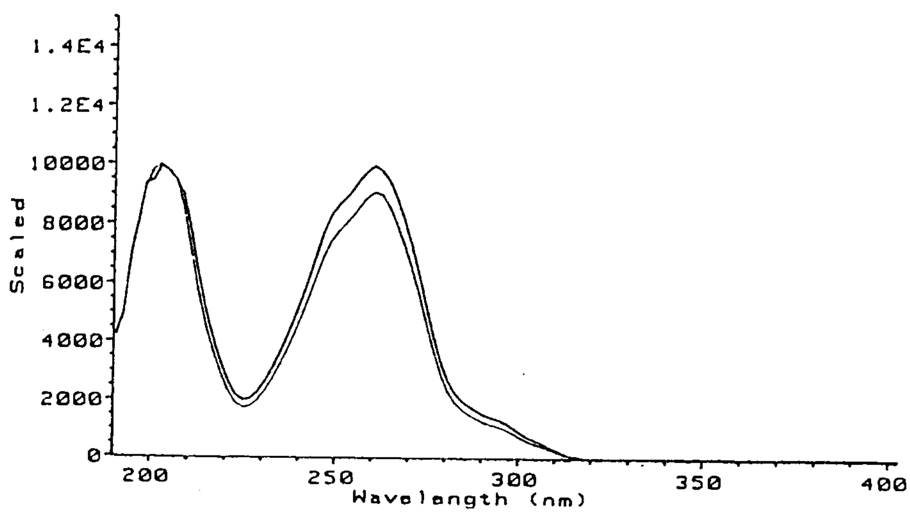


FIG 24

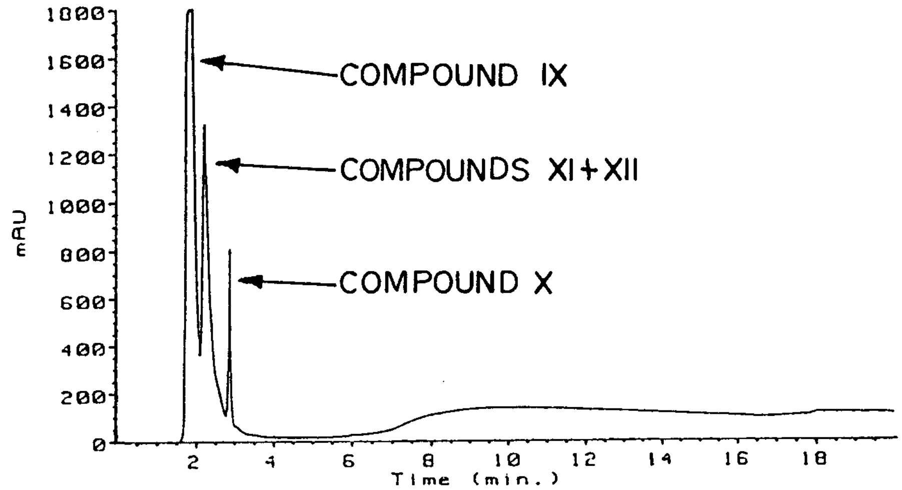


FIG 25

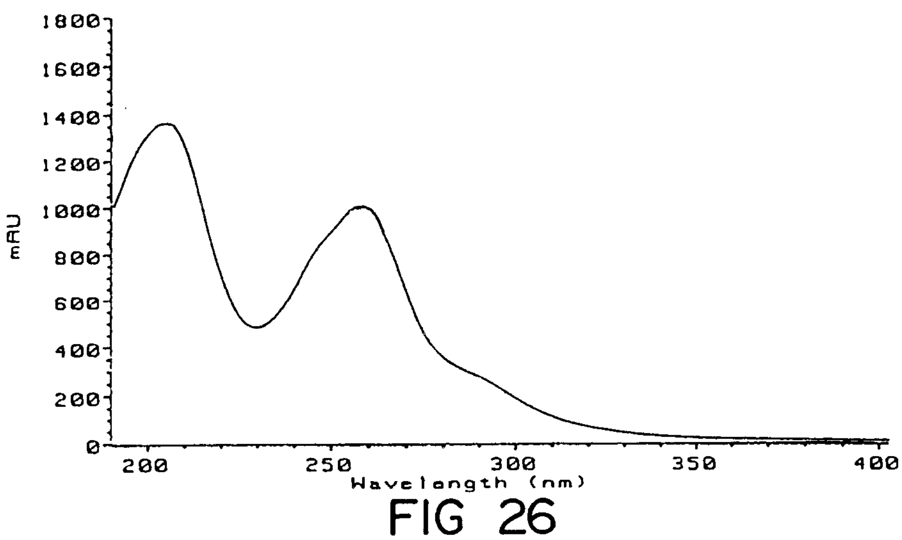


FIG 26

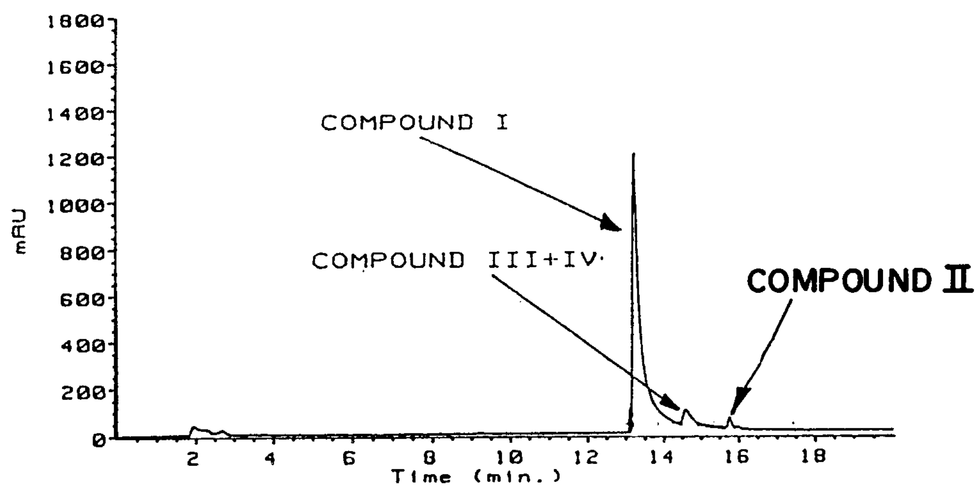


FIG 27

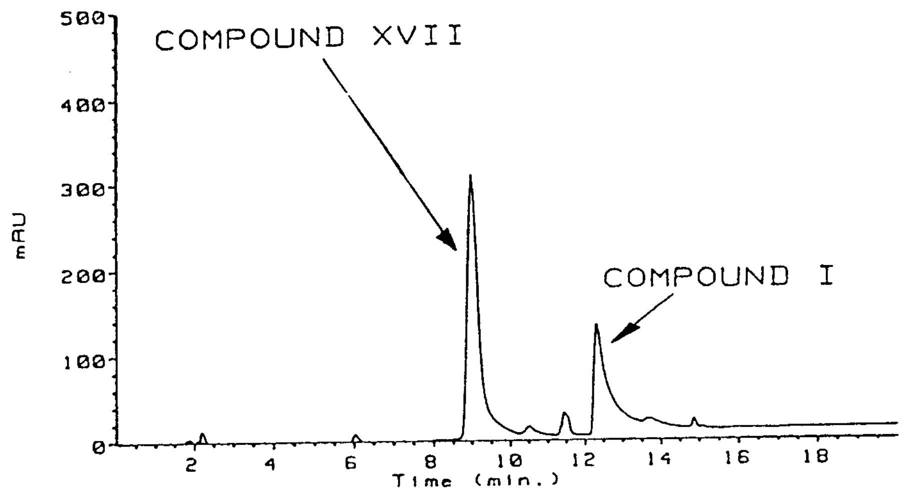


FIG 28

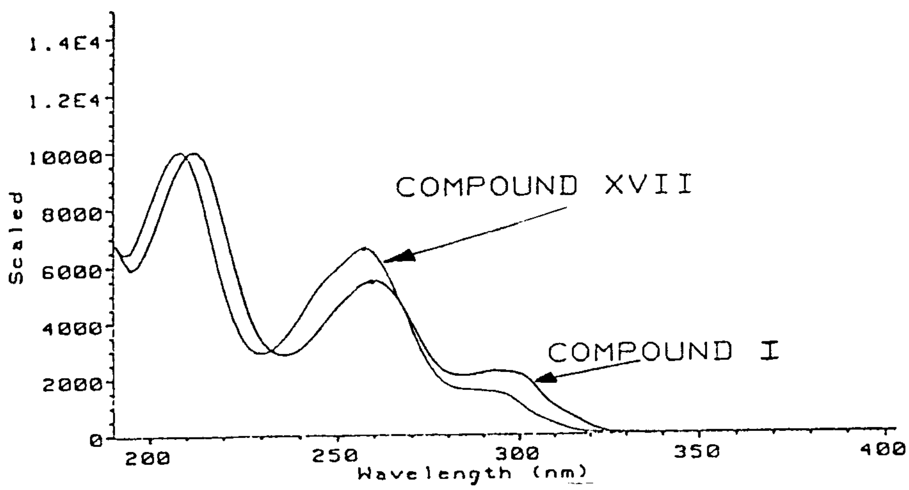


FIG 29

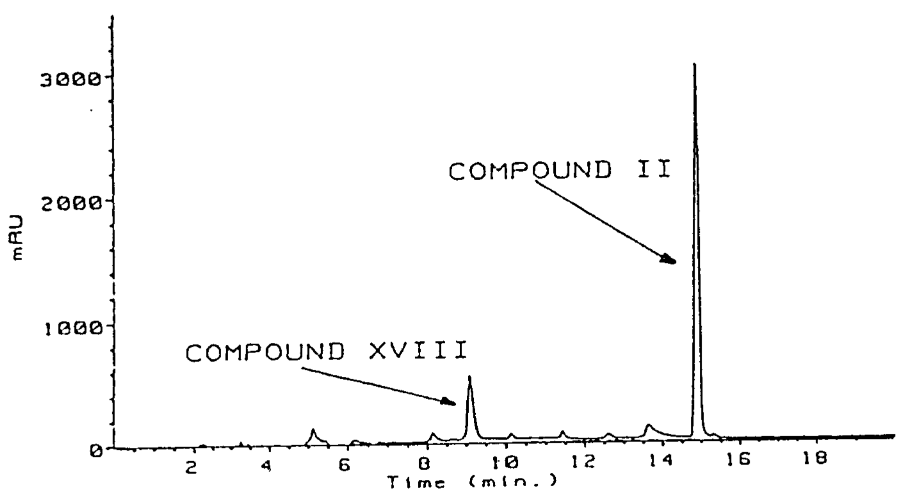


FIG 30

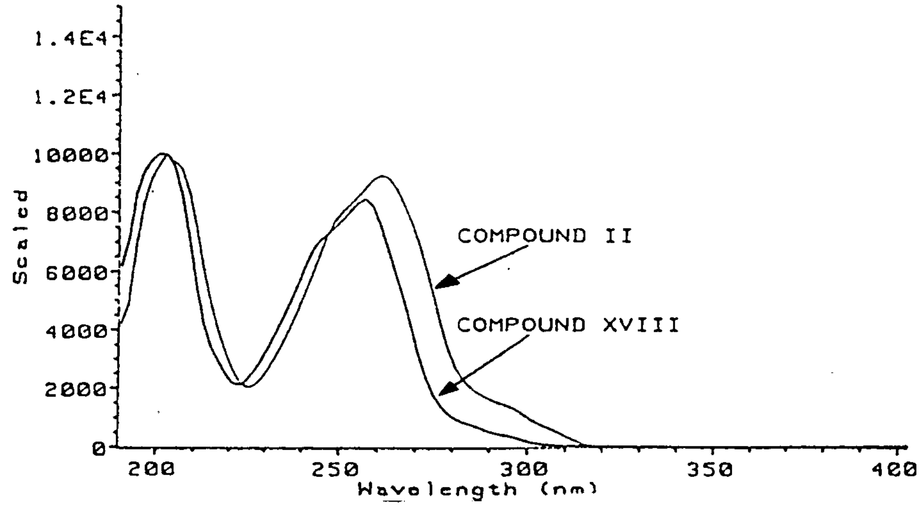
1.4E4
1.2E4
10000
8000
6000
4000
2000
0
Scaled
200
250
300
350
400
Wavelength (nm)
COMPOUND II
COMPOUND XVIII

FIG 31

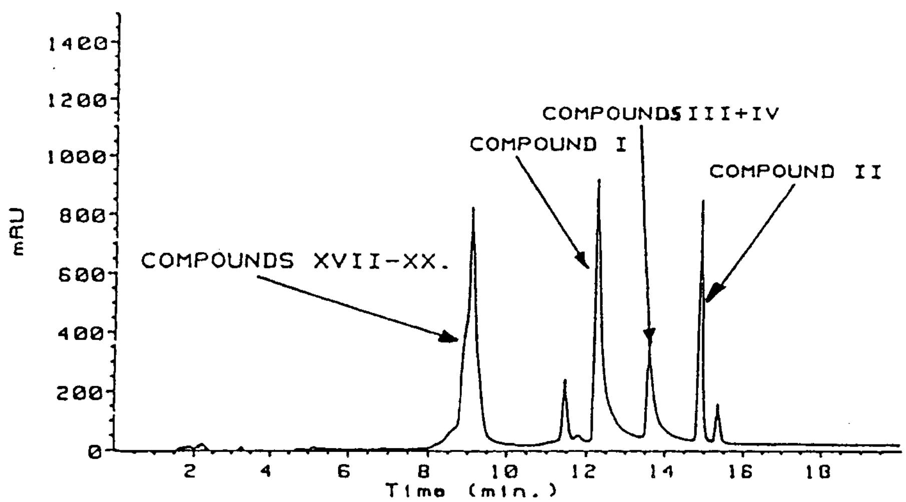
1400
1200
1000
800
600
400
200
0
mAU
2
4
6
8
10
12
14
16
18
Time (min.)
COMPOUNDS XVII-XX.
COMPOUND I
COMPOUNDS III+IV
COMPOUND II

FIG 32

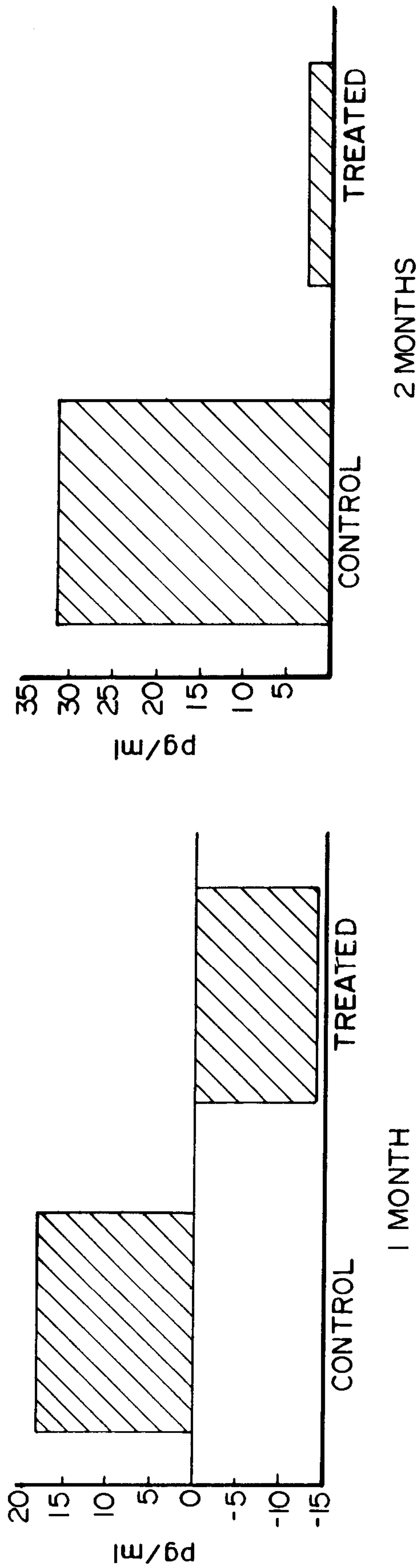
pg/ml
20
15
10
5
0
-5
-10
-15
CONTROL
TREATED
1 MONTH
pg/ml
35
30
25
20
15
10
5
CONTROL
TREATED
2 MONTHS

FIG 33

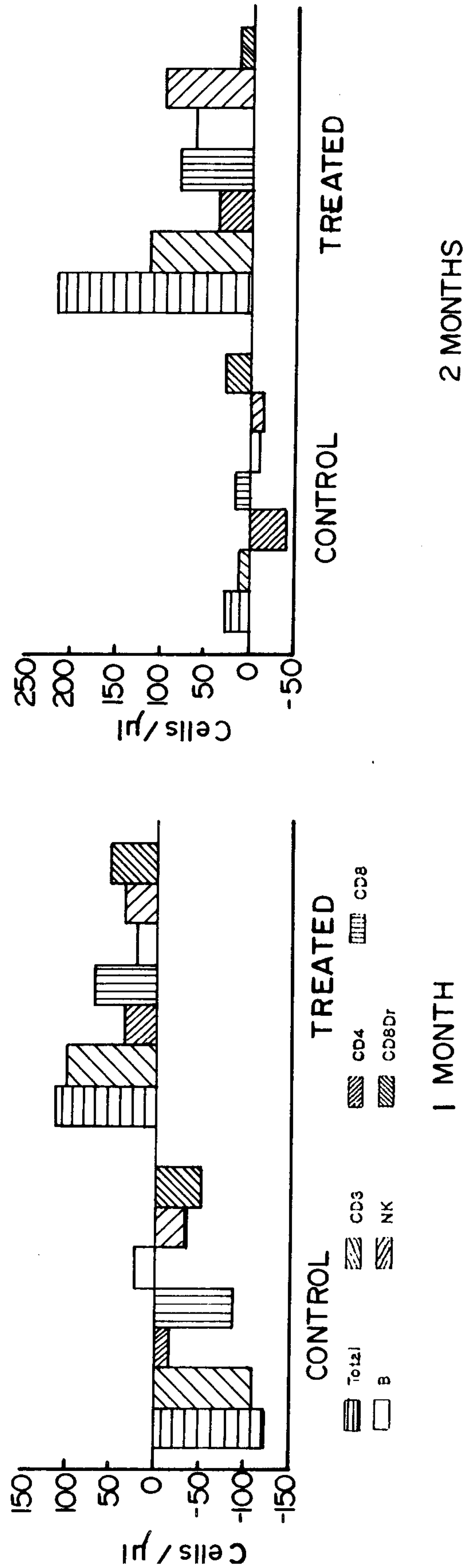
Cells / µl
150
100
50
0
-50
-100
-150
CONTROL
TREATED
Total
B
CD3
NK
CD4
CD8Dr
CD8
1 MONTH
Cells / µl
250
200
150
100
50
0
-50
CONTROL
TREATED
2 MONTHS

FIG 34

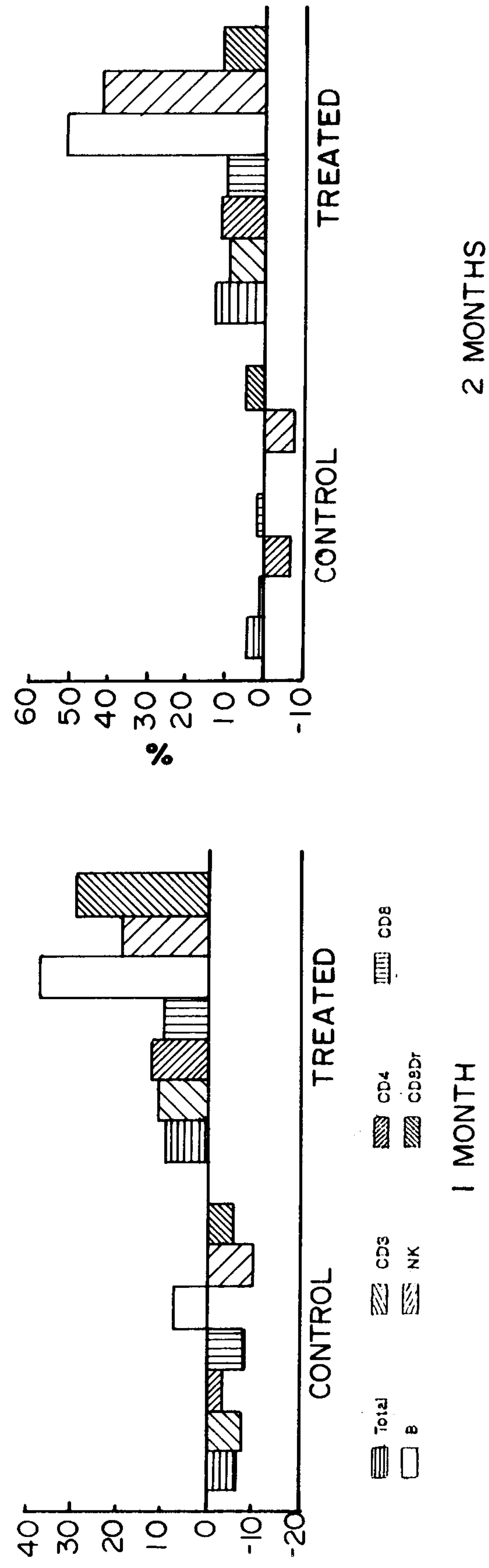
%
60
50
40
30
20
10
0
-10
CONTROL
TREATED
2 MONTHS
%
40
30
20
10
0
-10
-20
CONTROL
TREATED
Total
B
CD3
NK
CD4
CD8Dr
CD8
1 MONTH

FIG 35

European Patent Office

# EUROPEAN SEARCH REPORT

Application Number
EP 93 30 7030

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | EP-A-0 206 765 (ROOPEROL NV) 30 December 1986 --- | | A61K31/225<br>A61K31/70 |
| A | EP-A-0 130 829 (ROOPEROL NV) 9 January 1985 --- | | |
| A | GB-A-2 120 650 (ROOPEROL NV) 7 December 1983 --- | | |
| A | PLANT CELL TISSUE ORGAN CULT.<br>vol. 9, no. 2 , 1987<br>pages 131 - 136<br>Y.M. PAGE ET AL 'Hypoxoside production in tissue cultures of Hypoxis rooperi.' --- | | |
| A | J. BIOPHARM. SCI.<br>vol. 2, no. 4 , 1991<br>pages 305 - 318<br>H.P. KOCH ET AL. 'Biopharmaceutical investigation of hypoxoside, a natural antitumour compound from plant origin.' ----- | | TECHNICAL FIELDS SEARCHED (Int.Cl.5)<br>A61K |

The present search report has been drawn up for all claims

| Place of search | Date of completion of the search | Examiner |
|---|---|---|
| THE HAGUE | 12 November 1993 | KLAVER, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)